# EUROPEAN PATENT SPECIFICATION

(11) **EP 2 780 000 B1**
(45) Date of publication and mention of the grant of the patent: **03.04.2019**
(21) Application number: 12787711.6
(22) Date of filing: 15.11.2012
(51) Int. Cl.: A61K 9/20, A61K 31/485

(54) **TAMPER-RESISTANT ORAL PHARMACEUTICAL DOSAGE FORM COMPRISING A PHARMACOLOGICALLY ACTIVE INGREDIENT, AN OPIOID ANTAGONIST AND/OR AVERSIVE AGENT, POLYALKYLENE OXIDE AND ANIONIC POLYMER**
GEGEN MISSBRAUCH GESICHERTE ORALE PHARMAZEUTISCHE DOSISFORM, DIE EINEN PHARMAKOLOGISCH AKTIVEN WIRKSTOFF, EINEN OPIOID-ANTAGONISTEN ODER ABSCHRECKUNGSMITTEL, POLYALKYLENOXID UND EIN ANIONISCHES POLYMER UMFASST
FORME PHARMACEUTIQUE ORALE INVIOLABLE COMPORTANT UN AGENT PAHRMACOLOGIQUEMENT ACTIF, UN ANTAGONISTE OPIOÏDE OU AGENT DISSUASIF, UN OXYDE DE POLYALKYLÈNE ET UN POLYMÈRE ANIONIQUE

(30) Priority: 17.11.2011 EP 11009129; 28.02.2012 EP 12001295
(43) Date of publication of application: 24.09.2014
(73) Proprietor: Grünenthal GmbH, 52078 Aachen (DE)
(72) Inventor: GEISSLER, Anja, 52222 Stolberg (DE); BARNSCHEID, Lutz, 41239 Mönchengladbach (DE); SCHWIER, Sebastian, 52076 Aachen (DE); BARTHOLOMÄUS, Johannes, 52080 Aachen (DE)
(74) Representative: Bülle, Jan
(86) International application number: PCT/EP2012/072678
(87) International publication number: WO 2013/072395

(56) References cited:
- EP-A2- 1 842 533
- WO-A2-2010/140007
- DE-A1-102007 011 485
- US-A- 5 866 164
- "Note for guidance on stability testing EMeA", , 1 August 2003 (2003-08-01), XP055019638, Retrieved from the Internet: URL:http://www.emea.europa.eu/docs/en_GB/d ocument_library/Scientific_guideline/2009/ 09/WC500002651.pdf [retrieved on 2012-02-16]

## Description

The invention relates to a pharmaceutical dosage form for oral administration having a breaking strength of at least 300 N and comprising (i) a pharmacologically active ingredient, being an opioid agonist; (ii) an opioid antagonist; (iii) a polyalkylene oxide having an average molecular weight of at least 200,000 g/mol; and comprising (iv) an anionic polymer.

Tamper-resistant pharmaceutical dosage forms containing opioid agonists have been known for many years. Some concepts of rendering pharmaceutical dosage forms tamper resistant rely on the presence of opioid antagonists.

In some embodiments, the opioid agonist is provided in releasable form and the opioid antagonist is sequestered and not released when the pharmaceutical dosage form is administered in the prescribed manner, i.e. intact and orally. Only when the pharmaceutical dosage form is tampered with, e.g. by mechanical disruption such as pulverization, the opioid antagonist is released from the pharmaceutical dosage form thereby evolving its antagonizing effect and avoiding misuse of the opioid agonist.

In other embodiments, the opioid antagonist is released from the pharmaceutical dosage form upon prescribed administration, e.g. oral administration, but due to its chemical nature, pharmacokinetic properties, and pharmacodynamic properties, the antagonizing effect of the opioid antagonist does not evolve. This can be achieved by employing opioid antagonists that have no or only a very poor bioavailability when being administered by the prescribed route, e.g. orally. Only when the pharmaceutical dosage form is tampered with, e.g. by liquid extraction of the constituents and administration of the liquid extract by another route, typically parenterally such as intravenously, the opioid antagonist has a sufficient bioavailability so that it evolves its antagonizing effects and can avoid misuse of the opioid agonist.

EP 1 492 506 discloses pharmaceutical dosage forms containing a combination of the opioid agonist oxycodone and the opioid antagonist naloxone which are both embedded in a lipophilic matrix of ethylcellulose. Pharmaceutical dosage forms of this type are currently commercialized as Targin®. Abuse by intravenous administration can be prevented by the opioid antagonist naloxone. However, the dosage form can be misused by oral administration, as naloxone is not bioavailable upon oral administration. Further, misuse of the dosage form cannot be prevented either. The breaking strength of the dosage forms is far below 300 N.

US 5 866 164 relates to osmotic dosage forms comprising an opioid analgesic, an opioid antagonist and a high molecular weight poly(alkylene) or a poly(carboxymethylcellulose).

WO 2010/140007 discloses a tamper resistant dosage form comprising melt-extruded particulates which are present as a discontinuous phase in a matrix. The particulates comprise a drug, e.g. an opioid agonist and optionally an opioid antagonist.

Other concepts of rendering pharmaceutical dosage forms tamper resistant rely on the mechanical properties of the pharmaceutical dosage forms, particularly a substantially increased breaking strength (resistance to crushing). The major advantage of such pharmaceutical dosage forms is that comminuting, particularly pulverization, by conventional means, such as grinding in a mortar or fracturing by means of a hammer, is impossible or at least substantially impeded. Thus, by conventional means that are available to an abuser, such pharmaceutical dosage forms cannot be converted into a form suitable for abuse, e.g. a powder for nasal administration.

Such pharmaceutical dosage forms may additionally contain aversive agents such as opioid antagonists, which are locally separated from the opioid agonist in the pharmaceutical dosage form, i.e. the pharmaceutical dosage forms comprise subunits containing opioid agonist but no opioid antagonist, and other subunits containing opioid antagonist but no opioid agonist. When these pharmaceutical dosage forms are administered in a prescribed manner, the opioid antagonist is not released from the pharmaceutical dosage form and thus, does not exhibit any effect. In this regard it can be referred to e.g., WO 2005/016313, WO 2005/016314, WO 2005/ 063214, WO 2005/102286, WO 2006/002883, WO 2006/002884, WO 2006/002886, WO 2006/082097, WO 2006/082099, and WO 2008/107149.

EP 1 897 545 discloses pharmaceutical dosage forms containing the opioid agonist oxycodone which is embedded in a hydrophilic matrix of polyethylene oxide. The dosage forms are manufactured by compression of a powder mixture and subsequent heating. Pharmaceutical dosage forms of this type are currently commercialized as Oxycontin®. These dosage forms do not contain an opioid antagonist.

The known tamper resistant pharmaceutical dosage forms are not satisfactory in every respect. Manufacture is complicated and laborious, as different subunits need to be prepared separately and are mixed with one another subsequently, before the final pharmaceutical dosage form is formed. Under these circumstances, content uniformity and other requirements are difficult to satisfy. Further, the release profile of the opioid agonist typically differs from that of the opioid antagonist. This is because due to their different chemical nature, the dispersibility of the opioid agonist in the other excipients of the pharmaceutical dosage form typically differs from the dispersibility of the opioid antagonist. The same applies to their solubility in the release medium. Furthermore, storage stability and shelf-life of the dosage forms need to be improved.

There is a demand for tamper resistant pharmaceutical dosage forms that contain opioid agonists and that have advantages compared to the pharmaceutical dosage forms of the prior art.

This object has been achieved by the subject-matter of the patent claims.

A first aspect of the invention relates to a pharmaceutical dosage form for oral administration having a breaking strength of at least 300 N and comprising (i) a pharmacologically active ingredient, being an opioid agonist; (ii) an opioid antagonist; (iii) a polyalkylene oxide having an average molecular weight of at least 200,000 g/mol; and comprising (iv) an anionic polymer.

It has been surprisingly found that the following objects concerning tamper resistance can be achieved simultaneously by means of the pharmaceutical dosage form containing an opioid antagonist according to the invention:
- when the pharmaceutical dosage form is not tampered with and is administered by the prescribed oral route, the opioid agonist develops its desired pharmacological effect and the opioid antagonist, which is simultaneously released, does not counter this effect of the opioid agonist, especially as the opioid antagonist is preferably very poorly or not bioavailable when being administered orally. Nevertheless, in the intestine the orally administered opioid antagonist can locally block the opioid receptors thereby preventing obstipation, an undesired adverse event otherwise occurring due to induction by the opioid agonist;
- when the pharmaceutical dosage form is tampered with by liquid extraction of the active ingredients and is then administered by the non-prescribed, parenteral route, the opioid antagonist is fully bioavailable and thus, fully develops its antagonizing effect thereby avoiding misuse of the opioid agonist;
- when attempts are made to mechanically disrupt the pharmaceutical dosage form by conventional means typically available to an abuser, particularly in order to prepare a powder that is suitable for e.g. nasal administration, such attempts fail due to the increased breaking strength of the pharmaceutical dosage form.

Further, it has been surprisingly found that the following objects concerning tamper resistance can be achieved simultaneously by means of the pharmaceutical dosage form containing an aversive agent according to the invention:
- when the pharmaceutical dosage form is not tampered with and is administered by the prescribed oral route, the pharmacologically active ingredient, being an opioid agonist develops its desired pharmacological effect and the aversive agent does not counter this effect of the pharmacologically active ingredient, being an opioid agonist;
- when the pharmaceutical dosage form is tampered with by liquid extraction of the active ingredients and is then administered by the non-prescribed, parenteral route, the aversive agent fully develops its aversive effect thereby avoiding misuse of the pharmacologically active ingredient, being an opioid agonist;
- when attempts are made to mechanically disrupt the pharmaceutical dosage form by conventional means typically available to an abuser, particularly in order to prepare a powder that is suitable for e.g. nasal administration, such attempts fail due to the increased breaking strength of the pharmaceutical dosage form.

Still further it has been surprisingly found that the shelf-life and storage stability of the pharmaceutical dosage forms can be improved by adding an anionic polymer. Furthermore, it has been surprisingly found that a certain quantity of polyalkylene oxide, on which the increased breaking strength of the pharmaceutical dosage form typically rely, can be replaced by anionic polymer without deteriorating the mechanical properties and the tamper resistance of the pharmaceutical dosage form.

The dosage form according to the invention is suitable for avoiding or at least substantially impeding oral abuse, dose dumping by means of alcoholic liquids and unintentional misuse.

The pharmaceutical dosage form according to the invention comprises
(i) a pharmacologically active ingredient;
(ii) an opioid antagonist; and
(iii) a polyalkylene oxide having an average molecular weight of at least 200,000 g/mol.

Furthermore,
- the pharmaceutical dosage form additionally comprises (iv) an anionic polymer.

The pharmaceutical dosage form according to the invention comprises an opioid antagonist.

The pharmaceutical dosage form contains an opioid antagonist. Under these circumstances, the pharmacologically active ingredient is an opioid agonist.

Preferably, the pharmacologically active ingredient, being an opioid agonist and the opioid antagonist are homogeneously distributed over the pharmaceutical dosage form or, when the pharmaceutical dosage form comprises a film coating, over the coated core of the pharmaceutical dosage form. Preferably, the pharmacologically active ingredient, being an opioid agonist and the opioid antagonist are intimately mixed with one another and homogeneously dispersed in the polyalkylene oxide and the anionic polymer, preferably in molecular disperse form.

Preferably, the pharmacologically active ingredient, being an opioid agonist is not locally separated from the opioid antagonist. Preferably, the pharmaceutical dosage form contains neither any subunits comprising pharmacologically active ingredient, being an opioid agonist but no opioid antagonist, nor any subunits comprising opioid antagonist but no pharmacologically active ingredient, being an opioid agonist.

The pharmacologically active ingredient, being an opioid agonist and the opioid antagonist are embedded in a prolonged release matrix comprising the polyalkylene oxide and the anionic polymer. Thus, the prolonged release matrix is preferably a hydrophilic matrix. It has been surprisingly found that the release of the pharmacologically active ingredient, being an opioid agonist and the opioid antagonist from the prolonged release matrix relies on a combined mechanism that is regulated by erosion and diffusion of the release medium into the matrix.

Preferably, the pharmacologically active ingredient, which is an opioid agonist, the opioid antagonist, the polyalkylene oxide and the anionic polymer are intimately mixed with one another forming a homogeneous mixture.

Preferably, the release profile of the pharmacologically active ingredient, being an opioid agonist is matrix-retarded. Preferably, the pharmacologically active ingredient, being an opioid agonist is embedded in a matrix comprising the polyalkylene oxide and the anionic polymer, said matrix controlling the release of the pharmacologically active ingredient, being an opioid agonist from the pharmaceutical dosage form.

Physiologically acceptable materials which are known to the person skilled in the art may be used as supplementary matrix materials. Polymers, particularly preferably cellulose ethers and/or cellulose esters are preferably used as hydrophilic matrix materials. Ethylcellulose, hydroxypropylmethylcellulose, hydroxypropylcellulose, hydroxymethylcellulose, hydroxyethylcellulose, and/or the derivatives thereof, such as the salts thereof are very particularly preferably used as matrix materials.

It has been surprisingly found that the release of both, the pharmacologically active ingredient, being an opioid agonist and the opioid antagonist, from the prolonged release matrix is substantially independent from the pH value of the release medium.

In a preferred embodiment, the pharmaceutical dosage form according to the invention is adapted for administration once daily. In another preferred embodiment, the pharmaceutical dosage form according to the invention is adapted for administration twice daily. In still another preferred embodiment, the pharmaceutical dosage form according to the invention is adapted for administration thrice daily, four times daily, five times daily, six times daily, or even more frequently.

For the purpose of the specification, "twice daily" means equal or nearly equal time intervals, i.e., about every 12 hours, or different time intervals, e.g., 8 and 16 hours or 10 and 14 hours, between the individual administrations.

For the purpose of the specification, "thrice daily" means equal or nearly equal time intervals, i.e., about every 8 hours, or different time intervals, e.g., 6, 6 and 12 hours; or 7, 7 and 10 hours, between the individual administrations.

In a preferred embodiment, in accordance with Ph. Eur., the *in vitro* release profile of the pharmacologically active ingredient, being an opioid agonist essentially corresponds to, i.e. is essentially identical to or at least resembling with the *in vitro* release profile of the opioid antagonist. For the purpose of the specification, "essentially corresponds" preferably means that pharmacologically active ingredient, being an opioid agonist and opioid antagonist are released according to same order kinetics, preferably both according to a prolonged release profile; preferably, however, "essentially corresponds" does not encompass pharmaceutical dosage forms where one of the pharmacologically active ingredient, being an opioid agonist and the opioid antagonist is released immediately, and the other one is released in a prolonged fashion.

It has been surprisingly found that an essentially identical or at least resembling *in vitro* release profile of pharmacologically active ingredient, being an opioid agonist and opioid antagonist can be achieved, though the pharmaceutical dosage form contains a polyalkylene oxide, in combination with an anionic polymer, i.e. hydrophilic polymers. Polyalkylene oxide is necessary in order to achieve the substantially increased breaking strength of at least 300 N of the pharmaceutical dosage form. It is known that pharmaceutical dosage forms containing tilidine as opioid agonist and naloxone as opioid antagonist embedded in a hydrophilic matrix do not provide such an essentially identical or at least resembling *in vitro* release profile of opioid agonist and opioid antagonist (cf. EP 1 492 506, paragraph [0026]). Rather, these pharmaceutical dosage forms exhibit an *in vitro* release profile of the opioid agonist that substantially differs from the *in vitro* release profile of the opioid antagonist. As it is desirable to have an essentially identical or at least resembling *in vitro* release profile of both, the pharmacologically active ingredient, being an opioid agonist and the opioid antagonist, attempts have been made in the art to somehow approximate both *in vitro* release profiles. This could be achieved on the basis of hydrophobic matrix materials which, however, are typically not suitable for manufacturing pharmaceutical dosage forms having an increased breaking strength of at least 300 N. It has now been surprisingly found that the same can be achieved even on the basis of a hydrophilic matrix material, namely polyalkylene oxide, preferably in combination with an anionic polymer, and optionally in combination with additional matrix polymers.

Preferably, at every point in time the *in vitro* release profile of the pharmacologically active ingredient, being an opioid agonist does absolutely not deviate by more than 10%, more preferably not more than 9%, still more preferably not more than 8%, yet more preferably not more than 7%, even more preferably not more than 6%, most preferably not more than 5% and in particular not more than 4% or not more than 3% from the *in vitro* release profile of the opioid antagonist. For example, if the pharmaceutical dosage form releases under *in vitro* conditions in accordance with Ph. Eur. 23% of the opioid antagonist 2 h after administration, it preferably releases 23±10% (= from 13% to 33%) of the pharmacologically active ingredient, being an opioid agonist 2 h after administration.

Preferably, the pharmaceutical dosage form according to the invention causes an at least partially delayed or prolonged release of pharmacologically active ingredient, being an opioid agonist and opioid antagonist.

Controlled or prolonged release is understood according to the invention preferably to mean a release profile in which the pharmacologically active ingredient, being an opioid agonist and the opioid antagonist is released over a relatively long period with reduced intake frequency with the purpose of extended therapeutic action of the pharmacologically active ingredient, being an opioid agonist. Preferably, the meaning of the term "prolonged release" is in accordance with the European guideline on the nomenclature of the release profile of pharmaceutical dosage forms (CHMP). This is achieved in particular with peroral administration. The expression "at least partially delayed or prolonged release" covers according to the invention any pharmaceutical dosage forms which ensure modified release of the pharmacologically active ingredient, being opioid agonists and opioid antagonists contained therein. The pharmaceutical dosage forms preferably comprise coated or uncoated pharmaceutical dosage forms, which are produced with specific auxiliary substances, by particular processes or by a combination of the two possible options in order purposefully to change the release rate or location of release.

In the case of the pharmaceutical dosage forms according to the invention, the release profile of a controlled release form may be modified e.g. as follows: extended release, repeat action release, prolonged release and sustained release.

For the purpose of the specification "controlled release" preferably means a product in which the release of active compound over time is controlled by the type and composition of the formulation. For the purpose of the specification "extended release" preferably means a product in which the release of active compound is delayed for a finite lag time, after which release is unhindered. For the purpose of the specification "repeat action release" preferably means a product in which a first portion of active compound is released initially, followed by at least one further portion of active compound being released subsequently. For the purpose of the specification "prolonged release" preferably means a product in which the rate of release of active compound from the formulation after administration has been reduced over time, in order to maintain therapeutic activity, to reduce toxic effects, or for some other therapeutic purpose. For the purpose of the specification "sustained release" preferably means a way of formulating a medicine so that it is released into the body steadily, over a long period of time, thus reducing the dosing frequency. For further details, reference may be made, for example, to K.H. Bauer, Lehrbuch der Pharmazeutischen Technologie, 6th edition, WVG Stuttgart, 1999; and Eur. Ph.

The pharmaceutical dosage form according to the invention may comprise one or more pharmacologically active ingredients, being opioid agonists and opioid antagonists at least in part in a further controlled release form, wherein controlled release may be achieved with the assistance of conventional materials and processes known to the person skilled in the art, for example by embedding the substances in a controlled release matrix or by applying one or more controlled release coatings. Substance release must, however, be controlled such that addition of delayed-release materials does not impair the necessary breaking strength. Controlled release from the pharmaceutical dosage form according to the invention is achieved by embedding the pharmacologically active ingredient, being an opioid agonist and the opioid antagonist in a matrix. The polyalkylene oxide and the anionic polymer serve as matrix material, optionally in combination with auxiliary substances also acting as matrix materials. The auxiliary substances acting as matrix materials control release. Matrix materials may, for example, be hydrophilic, gel-forming materials, from which release proceeds mainly by erosion and diffusion.

Preferably, the release profile is substantially matrix controlled, preferably by embedding the pharmacologically active ingredient, being an opioid agonist and the opioid antagonist in a matrix comprising the polyalkylene oxide, the anionic polymer and optionally, additional matrix materials. Preferably, the release profile is not osmotically driven. Preferably, release kinetics is not zero order.

In preferred embodiments, in accordance with Ph. Eur., the *in vitro* release profile of the pharmacologically active ingredient, being an opioid agonist or the *in vitro* release profile of the opioid antagonist, preferably the *in vitro* release profile of both, the pharmacologically active ingredient, being an opioid agonist as well as the opioid antagonist in each case complies with any same single one of the following release profiles R¹ to R⁵⁰:

| % | R¹ | R² | R³ | R⁴ | R⁵ | R⁶ | R⁷ | R⁸ | R⁹ | R¹⁰ |
|---|---|---|---|---|---|---|---|---|---|---|
| 1 h | 30±28 | 30±26 | 30±24 | 30±22 | 30±20 | 30±18 | 30±16 | 30±14 | 30±12 | 30±10 |
| 2 h | 45±40 | 45±38 | 45±36 | 45±34 | 45±32 | 45±30 | 45±28 | 45±26 | 45±24 | 45±24 |
| 4 h | 60±35 | 60±33 | 60±31 | 60±29 | 60±27 | 60±25 | 60±23 | 60±21 | 60±19 | 60±17 |
| 6 h | 70±30 | 70±28 | 70±25 | 70±23 | 70±21 | 70±19 | 70±17 | 70±15 | 70±13 | 70±11 |
| 8 h | ≥60 | 85±13 | 85±12 | 85±11 | 85±10 | 85±9 | 85±8 | 85±7 | 85±6 | 85±5 |
| 10 h | ≥70 | ≥72 | ≥74 | ≥76 | ≥78 | ≥80 | ≥82 | ≥84 | ≥86 | ≥88 |
| 12 h | ≥80 | ≥82 | ≥84 | ≥86 | ≥88 | ≥90 | ≥92 | ≥94 | ≥96 | ≥98 |

| % | R¹¹ | R¹² | R¹³ | R¹⁴ | R¹⁵ | R¹⁶ | R¹⁷ | R¹⁸ | R¹⁹ | R²⁰ |
|---|---|---|---|---|---|---|---|---|---|---|
| 1 h | 40±38 | 40±36 | 40±34 | 40±32 | 40±30 | 40±28 | 40±26 | 40±24 | 40±22 | 40±20 |
| 2 h | 55±43 | 55±41 | 55±39 | 55±37 | 55±35 | 55±33 | 55±31 | 55±29 | 55±27 | 55±25 |
| 4 h | 70±28 | 70±26 | 70±24 | 70±22 | 70±20 | 70±18 | 70±16 | 70±14 | 70±12 | 70±10 |
| 6 h | 80±20 | 80±18 | 80±16 | 80±15 | 80±14 | 80±13 | 80±12 | 80±11 | 80±10 | 80±9 |
| 8 h | ≥80 | 90±8 | 90±8 | 90±7 | 90±7 | 90±6 | 90±6 | 90±5 | 90±5 | 90±4 |
| 10 h | ≥85 | ≥87 | ≥89 | ≥90 | ≥90 | ≥91 | ≥91 | ≥92 | ≥92 | ≥92 |
| 12 h | ≥90 | ≥91 | ≥91 | ≥91 | ≥92 | ≥92 | ≥92 | ≥93 | ≥93 | ≥93 |

| % | R²¹ | R²² | R²³ | R²⁴ | R²⁵ | R²⁶ | R²⁷ | R²⁸ | R²⁹ | R³⁰ |
|---|---|---|---|---|---|---|---|---|---|---|
| 1 h | 20±18 | 20±16 | 20±14 | 20±13 | 20±12 | 20±11 | 20±10 | 20±9 | 20±8 | 20±7 |
| 2 h | 35±33 | 35±31 | 35±30 | 35±29 | 35±27 | 35±25 | 35±23 | 35±21 | 35±19 | 35±17 |
| 4 h | 50±48 | 50±46 | 50±44 | 50±42 | 50±40 | 50±38 | 50±36 | 50±34 | 50±32 | 50±31 |
| 6 h | 60±38 | 60±36 | 60±34 | 60±32 | 60±30 | 60±28 | 60±26 | 60±24 | 60±22 | 60±20 |
| 8 h | ≥60 | 70±28 | 70±26 | 70±24 | 70±22 | 70±20 | 70±18 | 70±16 | 70±14 | 70±12 |
| 10 h | ≥70 | ≥72 | ≥74 | ≥76 | ≥78 | ≥80 | ≥82 | ≥84 | ≥86 | ≥88 |
| 12 h | ≥80 | ≥82 | ≥84 | ≥86 | ≥88 | ≥90 | ≥91 | ≥92 | ≥93 | ≥93 |

| % | R³¹ | R³² | R³³ | R³⁴ | R³⁵ | R³⁶ | R³⁷ | R³⁸ | R³⁹ | R⁴⁰ |
|---|---|---|---|---|---|---|---|---|---|---|
| 1 h | 8±7 | 8±6 | 8±5 | 8±4 | 13±12 | 13±10 | 13±8 | 13±6 | 18±17 | 18±14 |
| 2 h | 15±14 | 15±11 | 15±8 | 15±5 | 24±23 | 24±18 | 24±13 | 24±8 | 33±32 | 33±24 |
| 4 h | 30±29 | 30±22 | 30±15 | 30±8 | 38±37 | 38±28 | 38±18 | 38±8 | 55±34 | 55±26 |
| 6 h | 50±49 | 50±37 | 50±25 | 50±13 | 60±39 | 60±29 | 60±19 | 60±9 | 70±29 | 70±22 |
| 8 h | 65±34 | 65±26 | 65±18 | 65±10 | 75±24 | 75±18 | 75±12 | 75±6 | 83±16 | 83±13 |
| 10 h | 85±14 | 85±11 | 85±8 | 85±5 | 87±12 | 87±10 | 87±8 | 87±6 | 90±9 | 90±8 |
| 12 h | >95 | >95 | >95 | >95 | >95 | >95 | >95 | >95 | >95 | >95 |

| % | R⁴¹ | R⁴² | R⁴³ | R⁴⁴ | R⁴⁵ | R⁴⁶ | R⁴⁷ | R⁴⁸ | R⁴⁹ | R⁵⁰ |
|---|---|---|---|---|---|---|---|---|---|---|
| 1 h | 18±11 | 18±8 | 25±24 | 25±18 | 25±12 | 25±6 | 40±39 | 40±29 | 40±19 | 40±9 |
| 2 h | 33±16 | 33±8 | 45±44 | 45±33 | 45±22 | 45±11 | 63±26 | 63±20 | 63±14 | 63±8 |
| 4 h | 55±18 | 55±10 | 70±29 | 70±22 | 70±15 | 70±8 | 85±14 | 85±12 | 85±10 | 85±8 |
| 6 h | 70±15 | 70±8 | 83±16 | 83±13 | 83±10 | 83±7 | 90±9 | 90±8 | 90±7 | 90±6 |
| 8 h | 83±10 | 83±7 | 92±7 | 92±6 | 92±6 | 92±5 | 92±7 | 92±7 | 92±6 | 92±6 |
| 10 h | 90±7 | 90±6 | 94±6 | 94±6 | 94±5 | 94±5 | 94±6 | 94±6 | 94±5 | 94±5 |
| 12 h | >95 | >95 | >95 | >95 | >95 | >95 | >95 | >95 | >95 | >95 |

Suitable *in vitro* conditions are known to the skilled artisan. In this regard it can be referred to, e.g., the Ph. Eur. Preferably, the *in vitro* release profile is measured under the following conditions: 600 ml of blank FeSSIF (pH 5.0) at temperature of 37°C with sinker (type 1 or 2). The rotation speed of the paddle is adjusted to 150/min. The pharmacologically active ingredient is detected by means of a spectrometric measurement with a wavelength of 218 nm.

Preferably, the release profile of the pharmaceutical dosage form according to the invention is stable upon storage, preferably upon storage at elevated temperature, e.g. 40°C, for 3 months in sealed containers. In this regard "stable" means that when comparing the initial release profile with the release profile after storage, at any given time point the release profiles deviate from one another absolutely by not more than 20%, more preferably not more than 15%, still more preferably not more than 10%, yet more preferably not more than 7.5%, most preferably not more than 5.0% and in particular not more than 2.5%.

Preferably, the pharmaceutical dosage form according to the invention is monolithic. Preferably, the pharmaceutical dosage form is a monolithic mass. The pharmaceutical dosage form is preferably prepared by thermoforming, particularly preferably by hot-melt extrusion. The melt extruded strands are preferably cut into monoliths, which are then preferably formed into tablets. In this regard, the term "tablets" is preferably not to be understood as pharmaceutical dosage forms being made by compression of powder or granules (*compressi*) but rather, as shaped extrudates.

In another preferred embodiment, the pharmaceutical dosage form according to the invention contains an aversive agent. Under these circumstances, the pharmacologically active ingredient is an opioid agonist.

Aversive agents are known to the skilled artisan and are to be understood as agents that impart an unpleasant (aversive) sensation to an abuser when the dosage form is tampered with so that tampering for the purpose of abusing the pharmacologically active ingredient, being an opioid agonist that is contained in the dosage form can be avoided or at least substantially impeded.

Preferred aversive agents include but are not limited to:
(a) substances which irritate the nasal passages and/or pharynx (in the following also referred to as "component (a)"),
(b) viscosity-increasing agents and/or gelling agents (in the following also referred to as "component (b)"),
(c) emetics (in the following also referred to as "component (c)"),
(d) dyes (in the following also referred to as "component (d)"),
(e) bitter substances (in the following also referred to as "component (e)"),
(f) surfactants (in the following also referred to as "component (f)"),
and combinations of any of the foregoing, including (a)+(b), (a)+(c), (a)+(d), (a)+(e), (a)+(f); (b)+(c), (b)+(d), (b)+(e), (b)+(f); (c)+(d), (c)+(e), (c)+(f); (d)+(e), (d)+(f); and (e)+(f).

Preferred ternary combinations include: (a)+(b)+(c), (a)+(b)+(d), (a)+(b)+(e), (a)+(b)+(f); (a)+(c)+(d), (a)+(c)+(e), (a)+(c)+(f); (a)+(d)+(e), (a)+(d)+(f); (a)+(e)+(f); (b)+(c)+(d), (b)+(c)+(f); (b)+(d)+(e), (b)+(d)+(f); (b)+(e)+(f); (c)+(d)+(e), (c)+(d)+(f); (c)+(e)+(f); (d)+(e)+(f).

In a preferred embodiment, the dosage form according to the invention comprises component (a), i.e. a substance which irritates the nasal passages and/or pharynx.

Preferred components (a), i.e. substances which irritate the nasal passages and/or pharynx according to the invention, are any substances which, when administered abusively via the nasal passages and/or pharynx, bring about a physical reaction which is either so unpleasant for the abuser that he/she does not wish to or cannot continue administration, for example burning, or physiologically counteracts taking of the corresponding opioid, for example due to increased nasal secretion or sneezing. These substances which conventionally irritate the nasal passages and/or pharynx may also bring about a very unpleasant sensation or even unbearable pain when administered parenterally, in particular intravenously, such that the abuser does not wish to or cannot continue taking the substance. Particularly suitable substances which irritate the nasal passages and/or pharynx are those which cause burning, itching, urge to sneeze, increased formation of secretions or a combination of at least two of these stimuli. Appropriate substances and the quantities thereof which are conventionally to be used are known per se to the person skilled in the art or may be identified by simple preliminary testing.

Component (a) is preferably based on one or more constituents or one or more plant parts of at least one hot substance drug. Corresponding hot substance drugs are known per se to the person skilled in the art and are described, for example, in "Pharmazeutische Biologie - Drogen und ihre Inhaltsstoffe" by Prof. Dr. Hildebert Wagner, 2nd. revised edition, Gustav Fischer Verlag, Stuttgart-New York, 1982, pages 82 et seq.

The dosage form obtained by the process according to the invention may preferably contain the plant parts of the corresponding hot substance drugs in a quantity of 0.01 to 30 wt.%, particularly preferably of 0.1 to 0.5 wt.%, in each case relative to the total weight of the dosage form. If one or more constituents of corresponding hot substance drugs are used, the quantity thereof in a dosage unit obtained by the process according to the invention preferably amounts to 0.001 to 0.005 wt.%, relative to the total weight of the dosage form.

One or more constituents of at least one hot substance drug selected from the group comprising Allii sativi bulbus (garlic), Asari rhizoma cum herba (Asarum root and leaves), Calami rhizoma (calamus root), Capsici fructus (capsicum), Capsici fructus acer (cayenne pepper), Curcumae longae rhizoma (turmeric root), Curcumae xanthorrhizae rhizoma (Javanese turmeric root), Galangae rhizoma (galangal root), Myristicae semen (nutmeg), Piperis nigri fructus (pepper), Sinapis albae semen (white mustard seed), Sinapis nigri semen (black mustard seed), Zedoariae rhizoma (zedoary root) and Zingiberis rhizoma (ginger root), particularly preferably from the group comprising Capsici fructus (capsicum), Capsici fructus acer (cayenne pepper) and Piperis nigri fructus (pepper) may preferably be contained as component (a) to the dosage form according to the invention.

The constituents of the hot substance drugs preferably comprise o-methoxy(methyl)phenol compounds, acid amide compounds, mustard oils or sulfide compounds or compounds derived therefrom. Particularly preferably, at least one constituent of the hot substance drugs is selected from the group consisting of myristicin, elemicin, isoeugenol, α-asarone, safrole, gingerols, xanthorrhizol, capsaicinoids, preferably capsaicin, capsaicin derivatives, such as N-vanillyl-9E-octadecenamide, dihydrocapsaicin, nordihydrocapsaicin, homocapsaicin, norcapsaicin and nomorcapsaicin, piperine, preferably trans-piperine, glucosinolates, preferably based on non-volatile mustard oils, particularly preferably based on p-hydroxybenzyl mustard oil, methylmercapto mustard oil or methylsulfonyl mustard oil, and compounds derived from these constituents.

In another preferred embodiment, the dosage form according to the invention comprises component (b), i.e. a viscosity-increasing agent and/or gelling agent, which, with the assistance of a necessary minimum quantity of an aqueous liquid, forms a gel with the extract obtained from the dosage form, which gel is virtually impossible to administer safely, and preferably remains visually distinguishable when introduced into a further quantity of an aqueous liquid.

For the purposes of the specification, visually distinguishable means that the opioid-containing gel formed with the assistance of a necessary minimum quantity of aqueous liquid, when introduced, preferably with the assistance of a hypodermic needle, into a further quantity of aqueous liquid at 37°C, remains substantially insoluble and cohesive and cannot straightforwardly be dispersed in such a manner that it can safely be administered parenterally, in particular intravenously. The material preferably remains visually distinguishable for at least one minute, preferably for at least 10 minutes.

The increased viscosity of the extract makes it more difficult or even impossible for it to be passed through a needle or injected. If the gel remains visually distinguishable, this means that the gel obtained on introduction into a further quantity of aqueous liquid, for example by injection into blood, initially remains in the form of a largely cohesive thread, which, while it may indeed be broken up mechanically into smaller fragments, cannot be dispersed or even dissolved in such a manner that it can safely be administered parenterally, in particular intravenously. Intravenous administration of such a gel would therefore most probably result in serious damage to the health of the abuser. In combination with at least one optionally present component (a) or (c) to (d), this additionally leads to unpleasant burning, vomiting, bad flavor and/or visual deterrence.

In order to verify whether a viscosity-increasing agent and/or gelling agent is suitable as component (b) in the dosage form according to the invention, the opioid is preferably mixed with the viscosity-increasing agent and suspended in 10 ml of water at a temperature of 25°C. If this results in the formation of a gel which fulfils the above-stated conditions, the corresponding viscosity-increasing agent is suitable for preventing or averting abuse of the dosage forms according to the invention.

Preferred viscosity-increasing agents and/or gelling agents include but are not limited to the group consisting of microcrystalline cellulose, e.g. with 11 wt.% carboxymethylcellulose sodium (Avicel® RC 591), carboxymethylcellulose sodium (Blanose®, CMC-Na C300P®, Frimulsion® BLC-5, Tylose® C300 P), locust bean flour (Cesagum® LA-200, Cesagum® LID/150, Cesagum® LN-1), pectins such as citrus pectin (Cesapectin® HM Medium Rapid Set), apple pectin, pectin from lemon peel, waxy maize starch (C*Gel® 04201), sodium alginate (Frimulsion® ALG (E401)), guar flour (Frimulsion® BM, Polygum® 26/1-75), iota carrageenan (Frimulsion® D021), karaya gum, gellan gum (Kelcogel® F, Kelcogel® LT100), galactomannan (Meyprogat® 150), tara stone flour (Polygum® 43/1), propylene glycol alginate (Protanal®-Ester SD-LB), sodium hyaluronate, tragacanth, tara gum (Vidogum® SP 200), fermented polysaccharide welan gum (K1A96), xanthan gum (Xantural® 180). The names stated in brackets are the trade names by which exemplified materials are known commercially. In general, a quantity of 0.1 to 5 wt.% of the viscosity-increasing agent(s) is sufficient to fulfill the above-stated conditions. Component (b), where provided, is preferably present in the dosage form according to the invention in quantities of ≥ 5 mg per dosage form.

In a particularly preferred embodiment, the viscosity-increasing agents and/or gelling agents that are present as component (b) are those which, on extraction from the dosage form with the necessary minimum quantity of aqueous liquid, form a gel which encloses air bubbles. The resultant gels are distinguished by a turbid appearance, which provides the potential abuser with an additional optical warning and discourages him/her from administering the gel parenterally.

It is also possible to formulate the viscosity-increasing agent and the other constituents in the dosage form according to the invention in a mutually spatially separated arrangement.

In still another preferred embodiment, the dosage form according to the invention comprises component (c), i.e. an emetic, which is preferably present in a spatially separated arrangement from the other components of the dosage form according to the invention and, when correctly used, is intended not to exert its effect in the body.

Suitable emetics for preventing abuse of an opioid are known to the person skilled in the art and may be present in the dosage form according to the invention as such or in the form of corresponding derivatives, in particular esters or ethers, or in each case in the form of corresponding physiologically acceptable compounds, in particular in the form of the salts or solvates thereof. An emetic based on one or more constituents of ipecacuanha (ipecac) root, preferably based on the constituent emetine may preferably be considered in the dosage form according to the invention, as are, for example, described in "Pharmazeutische Biologie - Drogen und ihre Inhaltsstoffe" by Prof. Dr. Hildebert Wagner, 2nd, revised edition, Gustav Fischer Verlag, Stuttgart, New York, 1982.

The dosage form according to the invention may preferably comprise the emetic emetine as component (c), preferably in a quantity of ≥ 10 mg, particularly preferably of ≥ 20 mg and very particularly preferably in a quantity of ≥ 40 mg per dosage form. Apomorphine may likewise preferably be used as an emetic for additional abuse-proofing, preferably in a quantity of preferably ≥ 3 mg, particularly preferably of ≥ 5 mg and very particularly preferably of ≥ 7 mg per administration unit.

In yet another preferred embodiment, the dosage form according to the invention comprises component (d), i.e. a dye, which brings about an intense coloration of a corresponding aqueous solution, in particular when the attempt is made to extract the opioid for parenteral, preferably intravenous administration, which coloration may act as a deterrent to the potential abuser. Suitable dyes and the quantities required for the necessary deterrence may be found e.g. in WO 03/015531.

In another preferred embodiment, the dosage form according to the invention comprises component (e), i.e. a bittering agent. The consequent impairment of the flavor of the dosage form additionally prevents oral and/or nasal abuse. Suitable bitter substances and the quantities effective for use may be found in US-2003/0064099 A1. Suitable bitter substances are preferably aromatic oils, preferably peppermint oil, eucalyptus oil, bitter almond oil, menthol, fruit aroma substances, preferably aroma substances from lemons, oranges, limes, grapefruit or mixtures thereof, and/or denatonium benzoate.

Preferred components (f), i.e. surfactants according to the invention, are nonionic, anionic or cationic surfactants. Ionic surfactants are particularly preferred. It has been found that surfactants can function as aversive agents when the opioid agonist is abused via a mucosa, e.g. nasally, resulting in an unpleasant burning sensation.

In a preferred embodiment, the surfactant has a HLB value (hydrophilic-lipophilic-balance) within the range of 10±9, more preferably 10±6, most preferably 10±3; or 15±9, more preferably 15±6, most preferably 15±3; or 20±9, more preferably 20±6, most preferably 20±3; or 25±9, more preferably 25±6, most preferably 25±3; or 30±9, more preferably 30±6, most preferably 30±3; or 35±9, more preferably 35±6, most preferably 35±3.

A preferred example of an anionic surfactant is sodium laurylsulfate.

Particularly when components (c) and/or (e) are contained in the dosage form according to the invention, care should taken to ensure that they are formulated in such a manner or are present in such a low dose that, when correctly administered, the dosage form is able to bring about virtually no aversive effect which impairs the patient or the efficacy of the opioid. If the dosage form according to the invention contains component (c) and/or (e), the dosage must be selected such that, when correctly orally administered, no negative effect is caused. If, however, the intended dosage of the dosage form is exceeded inadvertently, in particular by children, or in the event of abuse, nausea or an inclination to vomit or a bad flavor are produced. The particular quantity of component (c) and/or (e) which can still be tolerated by the patient in the event of correct oral administration may be determined by the person skilled in the art by simple preliminary testing.

If, however, irrespective of the fact that the dosage form according to the invention is virtually impossible to pulverize, the dosage form containing the components (c) and/or (e) is provided with protection, these components should preferably be used at a dosage which is sufficiently high that, when abusively administered, they bring about an intense aversive effect on the abuser.

This is preferably achieved by spatial separation of at least the opioid from components (c) and/or (e), wherein the opioid is present in at least one subunit (X) and components (c) and/or (e) is/are present in at least one subunit (Y), and wherein, when the dosage form is correctly administered, components (c) and (e) do not exert their effect on taking and/or in the body and the remaining components of the formulation are identical.

If the dosage form according to the invention comprises at least 2 of components (c) or (e), these may each be present in the same or different subunits (Y). Preferably, when present, all the components (c) and (e) are present in one and the same subunit (Y). For the purposes of the specification, subunits are solid formulations, which in each case, apart from conventional auxiliary substances known to the person skilled in the art, contain the opioid, preferably also at least the polyalkylene oxide and optionally at least one of the optionally present components (a) and/or (b) and/or (c) and/or (d) and/or (e) and/or (f).

One substantial advantage of the separated formulation of opioids from components (c) or (e) in subunits (X) and (Y) of the dosage form according to the invention is that, when correctly administered, components (c) and/or (e) are hardly released in the body or are released in such small quantities that they exert no effect which impairs the patient or therapeutic success or, on passing through the patient's body, they are only liberated in locations where they cannot be sufficiently absorbed to be effective. When the dosage form is correctly administered, preferably hardly any of components (c) and/or (e) is released into the patient's body or they go unnoticed by the patient. The person skilled in the art will understand that the above-stated conditions may vary as a function of the particular components (c) and/or (e) and of the formulation of the subunits or the dosage form. The optimum formulation for the particular dosage form may be determined by simple preliminary testing.

Should, contrary to expectations, the abuser succeed in comminuting such a dosage form according to the invention, which comprises components (c) and/or (d) and/or (e) and/or (f) in subunits (Y), for the purpose of abusing the opioid and obtain a powder which is extracted with a suitable extracting agent, not only the opioid but also the particular component (c) and/or (d) and/or (e) and/or (f) will be obtained in a form in which it cannot readily be separated from the opioid, such that when the dosage form which has been tampered with is administered, in particular by oral and/or parenteral administration, it will exert its effect on taking and/or in the body combined with an additional aversive effect on the abuser corresponding to component (c) and/or (e) or, when the attempt is made to extract the opioid, the coloration caused by component (d) will act as a deterrent and so prevent abuse of the dosage form.

A dosage form in which the opioid is spatially separated from components (c) and/or (d), preferably by formulation in different subunits, may be formulated according to the invention in many different ways, wherein the corresponding subunits of such a dosage form may each be present in any desired spatial arrangement relative to one another, provided that the above-stated conditions for the release of components (c) and/or (d) are fulfilled.

The person skilled in the art will understand that component(s) (a) and/or (b) and/or (f) which are optionally also present may preferably be formulated in the dosage form according to the invention both in the particular subunits (X) and (Y) and in the form of independent subunits corresponding to subunits (X) and (Y), provided that neither the abuse-proofing nor the opioid release in the event of correct administration is impaired by the nature of the formulation.

In a preferred embodiment of the dosage form according to the invention, subunits (X) and (Y) are present in multiparticulate form, wherein granules, spheroids, beads or pellets are preferred and the same form, i.e. shape, is selected for both subunit (X) and subunit (Y), such that it is not possible to separate subunits (X) from (Y) by mechanical selection. The multiparticulate forms are preferably of a size in the range from 0.1 to 3 mm, preferably of 0.5 to 2 mm. The subunits (X) and (Y) in multiparticulate form may also preferably be press-moulded into a tablet, wherein the final formulation in each case proceeds in such a manner that the subunits (X) and (Y) are also retained in the resultant dosage form. The multiparticulate subunits (X) and (Y) of identical shape should also not be visually distinguishable from one another so that the abuser cannot separate them from one another by simple sorting. This may, for example, be achieved by the application of identical coatings which, apart from this disguising function, may also incorporate further functions, such as, for example, delayed release of one or more opioids or provision of a finish resistant to gastric juices on the particular subunits.

In a further preferred embodiment of the present invention, subunits (X) and (Y) are in each case arranged in layers relative to one another. The layered subunits (X) and (Y) are preferably arranged for this purpose vertically or horizontally relative to one another in the dosage form according to the invention, wherein in each case one or more layered subunits (X) and one or more layered subunits (Y) may be present in the dosage form, such that, apart from the preferred layer sequences (X)-(Y) or (X)-(Y)-(X), any desired other layer sequences may be considered, optionally in combination with layers containing components (a) and/or (b).

Another preferred dosage form according to the invention is one in which subunit (Y) forms a core which is completely enclosed by subunit (X), wherein a separation layer (Z) may be present between said layers. Such a structure is preferably also suitable for the above-stated multiparticulate forms, wherein both subunits (X) and (Y) and an optionally present separation layer (Z), which should preferably satisfy the hardness requirement according to the invention, are then formulated in one and the same multiparticulate form using the process according to the invention.

In a further preferred embodiment of the dosage form according to the invention, the subunit (X) forms a core, which is enclosed by subunit (Y), wherein the latter comprises at least one channel which leads from the core to the surface of the dosage form.

The dosage form according to the invention may comprise, between one layer of the subunit (X) and one layer of the subunit (Y), in each case one or more, preferably one, optionally swellable separation layer (Z) which serves to separate subunit (X) spatially from (Y).

If the dosage form according to the invention comprises the layered subunits (X) and (Y) and an optionally present separation layer (Z) in an at least partially vertical or horizontal arrangement, the dosage form preferably takes the form of a tablet, a coextrudate or a laminate, which has been produced using the process according to the invention.

In one particularly preferred embodiment, the entirety of the free surface of subunit (Y) and optionally at least part of the free surface of subunit(s) (X) and optionally at least part of the free surface of the optionally present separation layer(s) (Z) may be coated with at least one barrier layer (Z') which prevents release of component (c) and/or (d) and/or (c) and/or (e) and/or (f). The barrier layer (Z') should preferably also fulfill the hardness conditions according to the invention.

Another particularly preferred embodiment of the dosage form according to the invention comprises a vertical or horizontal arrangement of the layers of subunits (X) and (Y) and at least one push layer (p) arranged there between, and optionally a separation layer (Z), in which dosage form the entirety of the free surface of the layer structure consisting of subunits (X) and (Y), the push layer and the optionally present separation layer (Z) is provided with a semipermeable coating (E), which is permeable to a release medium, i.e. conventionally a physiological liquid, but substantially impermeable to the opioid and to component (c) and/or (e), and wherein this coating (E) comprises at least one opening for release of the opioid in the area of subunit (X).

In a further preferred embodiment, the subunit (X) of the dosage form according to the invention is in the form of a tablet, the edge face and optionally one of the two main faces of which is covered with a barrier layer (Z') containing component (c) and/or (e).

The person skilled in the art will understand that the auxiliary substances of the subunit(s) (X) or (Y) and of the optionally present separation layer(s) (Z) and/or of the barrier layer(s) (Z') used in the production according to the invention of the respective dosage form will vary as a function of the arrangement thereof in the dosage form, the mode of administration and as a function of the particular opioid of the optionally present components (a) and/or (b) and/or (d) and of component (c) and/or (e). The materials which have the requisite properties are in each case known per se to the person skilled in the art.

If release of component (c) and/or (e) from subunit (Y) of the dosage form according to the invention is prevented with the assistance of a cover, preferably a barrier layer, the subunit may consist of conventional materials known to the person skilled in the art, preferably contain the polyalkylene oxide and preferably be produced according to the invention.

If a corresponding barrier layer (Z') is not provided to prevent release of component (c) and/or (e), the materials of the subunits should be selected such that release of the particular component (c) from subunit (Y) is virtually ruled out.

The materials which are stated below to be suitable for production of the barrier layer may preferably be used for this purpose and should preferably contain the polyalkylene oxide for fulfilling the hardness conditions.

Preferred materials are those which are selected from the group consisting of alkylcelluloses, hydroxyalkylcelluloses, glucans, scleroglucans, mannans, xanthans, copolymers of poly[bis(p-carboxyphenoxy)propane : sebacic acid], preferably in a molar ratio of 20:80 (marketed under the name Polifeprosan 20®), carboxymethylcelluloses, cellulose ethers, cellulose esters, nitrocelluloses, polymers based on (meth)acrylic acid and the esters thereof, polyamides, polycarbonates, polyalkylenes, polyalkylene glycols, polyalkylene oxides, polyalkylene terephthalates, polyvinyl alcohols, polyvinyl ethers, polyvinyl esters, halogenated polyvinyls, polyglycolides, polysiloxanes and polyurethanes and the copolymers thereof. Particularly suitable materials may be selected from the group consisting of methylcellulose, ethylcellulose, hydroxypropylcellulose, hydroxypropylmethylcellulose, hydroxybutylmethylcellulose, cellulose acetate, cellulose propionate (of low, medium or high molecular weight), cellulose acetate propionate, cellulose acetate butyrate, cellulose acetate phthalate, carboxymethylcellulose, cellulose triacetate, sodium cellulose sulfate, polymethyl methacrylate, polyethyl methacrylate, polybutyl methacrylate, polyisobutyl methacrylate, polyhexyl methacrylate, polyisodecyl methacrylate, polylauryl methacrylate, polyphenyl methacrylate, polymethyl acrylate, polyisopropyl acrylate, polyisobutyl acrylate, polyoctadecyl acrylate, polyethylene, low density polyethylene, high density polyethylene, polypropylene, polyethylene glycol, polyethylene oxide, polyethylene terephthalate, polyvinyl alcohol, polyvinyl isobutyl ether, polyvinyl acetate and polyvinyl chloride.

Particularly suitable copolymers may be selected from the group comprising copolymers of butyl methacrylate and isobutyl methacrylate, copolymers of methyl vinyl ether and maleic acid of high molecular weight, copolymers of methyl vinyl ether and maleic acid monoethyl ester, copolymers of methyl vinyl ether and maleic anhydride and copolymers of vinyl alcohol and vinyl acetate. Further materials which are particularly suitable for formulating the barrier layer are starch-filled polycaprolactone, aliphatic polyesteramides, aliphatic and aromatic polyester urethanes, polyhydroxyalkanoates, in particular polyhydroxybutyrates, polyhydroxy-valerates, casein, polylactides and copolylactides.

The above-stated materials may optionally be blended with further conventional auxiliary substances known to the person skilled in the art, preferably selected from the group consisting of glyceryl monostearate, semi-synthetic triglyceride derivatives, semi-synthetic glycerides, hydrogenated castor oil, glyceryl palmitostearate, glyceryl behenate, polyvinylpyrrolidone, gelatine, magnesium stearate, stearic acid, sodium stearate, talcum, sodium benzoate, boric acid and colloidal silica, fatty acids, substituted triglycerides, glycerides, polyoxyalkylene glycols and the derivatives thereof.

If the dosage form according to the invention comprises a separation layer (Z'), said layer, like the uncovered subunit (Y), may preferably consist of the above-stated materials described for the barrier layer. The person skilled in the art will understand that release of the opioid or of the aversive agent from the particular subunit may be controlled by the thickness of the separation layer.

The pharmaceutical dosage form according to the invention comprises a polyalkylene oxide having a weight average molecular weight Mw of at least 200,000 g/mol, preferably at least 500,000 g/mol, more preferably at least 750,000 g/mol, still more preferably at least 1,000,000 g/mol, yet more preferably at least 1,500,000 g/mol, most preferably at least 2,000,000 g/mol and in particular within the range of from 500,000 to 15,000,000 g/mol.

Preferably, the polyalkylene oxide is selected from the group consisting of polymethylene oxide, polyethylene oxide and polypropylene oxide, the copolymers and mixtures thereof.

Polyalkylene oxide may comprise a single polyalkylene oxide having a particular average molecular weight, or a mixture (blend) of different polymers, such as two, three, four or five polymers, e.g., polymers of the same chemical nature but different average molecular weight, polymers of different chemical nature but same average molecular weight, or polymers of different chemical nature as well as different molecular weight.

For the purpose of the specification, a polyalkylene glycol has a molecular weight of up to 20,000 g/mol whereas a polyalkylene oxide has a molecular weight of more than 20,000 g/mol. In a preferred embodiment, the weight average over all molecular weights of all polyalkylene oxides that are contained in the pharmaceutical dosage form is at least 200,000 g/mol. Thus, polyalkylene glycols, if any, are preferably not taken into consideration when determining the weight average molecular weight of polyalkylene oxide.

Preferably, the content of the polyalkylene oxide is within the range of from 20 to 99 wt.-%, more preferably 25 to 95 wt.-%, still more preferably 30 to 90 wt.-%, yet more preferably 30 to 85 wt.-%, most preferably 30 to 80 wt.-% and in particular 30 to 75 wt.-%, based on the total weight of the pharmaceutical dosage form.

In a preferred embodiment, the content of the polyalkylene oxide is at least 10 wt.-%, more preferably at least 15 wt.-%, still more preferably at least 20 wt.-%, yet more preferably at least 25 wt.-% and in particular at least 30 wt.-%, based on the total weight of the pharmaceutical dosage form.

In a preferred embodiment, the content of the polyalkylene oxide is at most 80 wt.-%, more preferably at most 75 wt.-%, still more preferably at most 70 wt.-%, yet more preferably at most 65 wt.-% and in particular at most 60 wt.-%, based on the total weight of the pharmaceutical dosage form. In another preferred embodiment, the content of the polyalkylene oxide is at most 55 wt.-%, more preferably at most 50 wt.-%, still more preferably at most 48 wt.-%, yet more preferably at most 45 wt.-% and in particular at most 42 wt.-%, based on the total weight of the pharmaceutical dosage form.

In a preferred embodiment, the overall content of polyalkylene oxide is within the range of 25±20 wt.-%, more preferably 25±15 wt.-%, most preferably 25±10 wt.-%, and in particular 25±5 wt.-%. In another preferred embodiment, the overall content of polyalkylene oxide is within the range of 35±20 wt.-%, more preferably 35±15 wt.-%, most preferably 35±10 wt.-%, and in particular 35±5 wt.-%. In a preferred embodiment, the overall content of polyalkylene oxide is within the range of 40±20 wt.-%, more preferably 40±15 wt.-%, most preferably 40±10 wt.-%, and in particular 40±5 wt.-%. In still another preferred embodiment, the overall content of polyalkylene oxide is within the range of 45±20 wt.-%, more preferably 45±15 wt.-%, most preferably 45±10 wt.-%, and in particular 45±5 wt.-%. In yet another preferred embodiment, the overall content of polyalkylene oxide is within the range of 55±20 wt.-%, more preferably 55±15 wt.-%, most preferably 55±10 wt.-%, and in particular 55±5 wt.-%. In a further preferred embodiment, the overall content of polyalkylene oxide is within the range of 65±20 wt.-%, more preferably 65±15 wt.-%, most preferably 65±10 wt.-%, and in particular 65±5 wt.-%. In still a further a preferred embodiment, the overall content of polyalkylene oxide is within the range of 75±20 wt.-%, more preferably 75±15 wt.-%, most preferably 75±10 wt.-%, and in particular 75±5 wt.-%. In a still further a preferred embodiment, the overall content of polyalkylene oxide is within the range of 80±15 wt.-%, more preferably 80±10 wt.-%, and most preferably 80±5 wt.-%.

Preferably, the relative weight ratio of the polyalkylene oxide to the opioid agonist is at least 0.5:1, more preferably at least 1:1, at least 2:1, at least 3:1, at least 4:1, at least 5:1, at least 6:1, at least 7:1, at least 8:1 or at least 9:1. In a preferred embodiment, the relative weight ratio of the polyalkylene oxide to the pharmacologically active ingredient, being an opioid agonist is within the range of from 5:1 to 1:1, more preferably 4:1 to 2:1.

In a preferred embodiment, the polyalkylene oxide is homogeneously distributed in the pharmaceutical dosage form according to the invention. Preferably, the polyalkylene oxide forms a matrix in which the pharmacologically active ingredient, being an opioid agonist and the opioid antagonist are embedded.

In a particularly preferred embodiment, the pharmacologically active ingredient, being an opioid agonist, the opioid antagonist, the polyalkylene oxide and the anionic polymer are intimately homogeneously distributed in the pharmaceutical dosage form so that the pharmaceutical dosage form does not contain any segments where either pharmacologically active ingredient, being an opioid agonist is present in the absence of opioid antagonist and/or polyalkylene oxide and/or anionic polymer, or where opioid antagonist is present in the absence of pharmacologically active ingredient, being an opioid agonist and/or polyalkylene oxide and/or anionic polymer or where polyalkylene oxide is present in the absence of pharmacologically active ingredient, being an opioid agonist and/or opioid antagonist and/or anionic polymer or where anionic polymer is present in the absence of pharmacologically active ingredient, being an opioid agonist and/or opioid antagonist and/or polyalkylene oxide.

When the pharmaceutical dosage form is film coated, the polyalkylene oxide is preferably homogeneously distributed in the core of the pharmaceutical dosage form, i.e. the film coating preferably does not contain polyalkylene oxide, but may e.g. contain polyethylene glycol. Nonetheless, the film coating as such may of course contain one or more polymers, which however, preferably differ from the polyalkylene oxide contained in the core.

The polyalkylene oxide may be combined with one or more additional polymers selected from the group consisting of polyalkylene oxide, preferably polymethylene oxide, polyethylene oxide, polypropylene oxide; polyethylene, polypropylene, polyvinyl chloride, polycarbonate, polystyrene, polyvinylpyrrolidone, poly(hydroxy fatty acids), such as for example poly(3-hydroxybutyrate-co-3-hydroxyvalerate) (Biopol®), poly(hydroxyvaleric acid); polycaprolactone, polyvinyl alcohol, polyesteramide, polyethylene succinate, polylactone, polyglycolide, polyurethane, polyamide, polylactide, polyacetal (for example polysaccharides optionally with modified side chains), polylactide/glycolide, polylactone, polyglycolide, polyorthoester, polyanhydride, block polymers of polyethylene glycol and polybutylene terephthalate (Polyactive®), polyanhydride (Polifeprosan), copolymers thereof, blockcopolymers thereof, and mixtures of at least two of the stated polymers, or other polymers with the above characteristics.

Preferably, the molecular weight dispersity Mw/Mn of polyalkylene oxide is within the range of 2.5±2.0, more preferably 2.5±1.5, still more preferably 2.5±1.0, yet more preferably 2.5±0.8, most preferably 2.5±0.6, and in particular 2.5±0.4.

The polyalkylene oxide preferably has a viscosity at 25°C of 30 to 17,600 cP, more preferably 55 to 17,600 cP, still more preferably 600 to 17,600 cP and most preferably 4,500 to 17,600 cP, measured in a 5 wt.-% aqueous solution using a model RVF Brookfield viscosimeter (spindle no. 2 / rotational speed 2 rpm); of 400 to 4,000 cP, more preferably 400 to 800 cP or 2,000 to 4,000 cP, measured on a 2 wt.-% aqueous solution using the stated viscosimeter (spindle no. 1 or 3 / rotational speed 10 rpm); or of 1,650 to 10,000 cP, more preferably 1,650 to 5,500 cP, 5,500 to 7,500 cP or 7,500 to 10,000 cP, measured on a 1 wt.-% aqueous solution using the stated viscosimeter (spindle no. 2 / rotational speed 2 rpm).

The pharmaceutical dosage form according to the invention, in addition to the polyalkylene oxide, contains an anionic polymer, which is preferably obtainable by polymerization of a monomer composition comprising an ethylenically unsaturated monomer bearing an anionic functional group, in protonated form or a physiologically acceptable salt thereof. Preferably, the anionic functional group is selected from carboxyl groups, sulfonyl groups, sulfate groups, and phosphoryl groups.

Preferably, the anionic polymer comprises at least 3 repeating units (e.g. monomeric units), more preferably at least 10 repeating units, still more preferably at least 100 repeating units, most preferably at least 1,000 repeating units and in particular at least 10,000 repeating units.

The pharmacologically active ingredient, being an opioid agonist and the opioid antagonist are then embedded in a controlled-release matrix comprising the polyalkylene oxide as well as said anionic polymer.

Preferably, the anionic polymer comprises anionic functional groups selected from carboxyl groups, sulfonyl groups, sulfate groups, and phosphoryl groups.

The anionic polymer is neither an anionic oligosaccharide nor an anionic polysaccharide. Examples of anionic oligosaccharides and anionic polysaccharides are derived from uronic acids. The anionic polymer is not a cellulose-derivative, such as carboxymethylcellulose sodium or croscarmellose sodium.

Preferably, the pharmaceutical dosage form does not contain any anionic oligosaccharide or anionic polysaccharide. More preferably, the pharmaceutical dosage form does not contain carboxymethylcellulose sodium or croscarmellose sodium.

The anionic polymer is derived from a monomer selected from acrylic acid, alkyl acrylates and alkyl alkacrylates, or a combination thereof.

Preferably, the anionic polymer is obtainable by polymerization of a monomer composition comprising an ethylenically unsaturated monomer selected from ethylenically unsaturated carboxylic acids, ethylenically unsaturated carboxylic acid anhydrides, ethylenically unsaturated sulfonic acids and mixtures thereof.

Preferred ethylenically unsaturated carboxylic acid and ethylenically unsaturated carboxylic acid anhydride monomers include the acrylic acids typified by acrylic acid itself, methacrylic acid, ethacrylic acid, alpha-chloracrylic acid, alpha-cyano acrylic acid, beta-methyl-acrylic acid (crotonic acid), alpha-phenyl acrylic acid, beta-acryloxy propionic acid, sorbic acid, alpha-chloro sorbic acid, angelic acid, cinnamic acid, p-chloro cinnamic acid, beta-styryl acrylic acid (1-carboxy-4-phenyl butadiene-1,3), itaconic acid, citraconic acid, mesaconic acid, glutaconic acid, aconitic acid, maleic acid, fumaric acid, tricarboxy ethylene and maleic acid anhydride.

Preferred ethylenically unsaturated sulfonic acids include aliphatic or aromatic vinyl sulfonic acids such as vinylsulfonic acid, allyl sulfonic acid, vinyltoluenesulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-acryloxy propyl sulfonic acid, 2-hydroxy-3-methacryloxy propyl sulfonic acid and 2-acrylamido-2-methyl propane sulfonic acid.

Preferably, the monomer composition comprises acrylic acid, methacrylic acid, and/or 2-acrylamido-2-methyl propane sulfonic acid. Acrylic acid is especially preferred.

The anionic polymer is obtainable by polymerization of such a monomer composition. This does not necessarily require that it has been obtained from such a monomer composition indeed. In other words, the anionic polymer is a polymer comprising at least one repeating unit which results from polymerization of an ethylenically unsaturated monomer bearing an anionic functional group, in protonated form or a physiologically acceptable salt thereof.

The anionic polymer may be linear or branched or cross-linked.

Preferably, anionic polymer is hydrophilic, more preferably water-soluble or water-swellable.

The anionic polymer may be a homopolymer or a copolymer. When anionic polymer is a homopolymer, it comprises a single type of repeating unit, i.e. is the polymerization product of a monomer composition comprising a single type of monomer. A homopolymer of acrylic acid, i.e. polyacrylic acid, is particularly preferred. When anionic polymer is a copolymer, it may comprise two, three or more different repeating units, i.e. may be the polymerization product of a monomer composition comprising two, three or more different monomers.

In a preferred embodiment, the anionic polymer is a copolymer, comprising from about 50 mol-% to 99.999 mol-%, and more preferably from about 75 mol-% to 99.99 mol-% repeating units bearing anionic functional groups, preferably acid groups, more preferably carboxylic groups.

Preferably, the anionic polymer has an average equivalent weight of 76±50 g/mol, more preferably of 76±30 g/mol, still more preferably of 76±20 g/mol and most preferably of 76±10 g/mol per carboxyl group.

In a preferred embodiment, the monomer composition from which anionic polymer is derivable, further comprises a cross-linking agent, i.e. in this embodiment the anionic polymer is cross-linked.

Suitable cross-linking agents include
- compounds having at least two polymerizable double bonds, e.g. ethylenically unsaturated functional groups;
- compounds having at least one polymerizable double bond, e.g. an ethylenically unsaturated functional group, and at least one functional group that is capable of reacting with another functional group of one or more of the repeating units of anionic polymer;
- compounds having at least two functional groups that are capable of reacting with other functional groups of one or more of the repeating units of anionic polymer; and
- polyvalent metal compounds which can form ionic cross-linkages, e.g. through the anionic functional groups.

Cross-linking agents having at least two polymerizable double bonds, preferably allyl groups, are particularly preferred.

Cross-linking agents having at least two polymerizable double bonds include (i) di- or polyvinyl compounds such as divinylbenzene and divinyltoluene; (ii) di- or poly-esters of unsaturated mono- or poly-carboxylic acids with polyols including, for example, di- or triacrylic acid esters of polyols such as ethylene glycol, trimethylol propane, glycerine, or polyoxyethylene glycols; (iii) bisacrylamides such as N,N-methylenebisacrylamide; (iv) carbamyl esters that can be obtained by reacting polyisocyanates with hydroxyl group-containing monomers; (v) di- or poly-allyl ethers of polyols; (vi) di- or poly-allyl esters of polycarboxylic acids such as diallyl phthalate, diallyl adipate, and the like; (vii) esters of unsaturated mono- or poly-carboxylic acids with mono-allyl esters of polyols such as acrylic acid ester of polyethylene glycol monoallyl ether; and (viii) di- or triallyl amine.

In a preferred embodiment, divinyl glycol (1,5-hexadiene-3,4-diol) is contained as cross-linking agent, whereas allyl or vinyl derivatives of polyols, such as allylsucrose or allyl pentaerythritol, are less preferred. This embodiment is preferably realized by polyacrylic acid polymers of polycarbophil type according to USP.

In another preferred embodiment, allyl derivatives of polyols, such as allylsucrose or allyl pentaerythritol, are contained as cross-linking agent, whereas divinyl glycol (1,5-hexadiene-3,4-diol) is less preferred. This embodiment is preferably realized by polyacrylic acid polymers of carbomer type according to USP or Ph. Eur.

Cross-linking agents having at least one polymerizable double bond and at least one functional group capable of reacting with other functional groups of one or more of the repeating units of anionic polymer include N-methylol acrylamide, glycidyl acrylate, and the like.

Suitable cross-linking agents having at least two functional groups capable of reacting with other functional groups of one or more of the repeating units of anionic polymer include glyoxal; polyols such as ethylene glycol; polyamines such as alkylene diamines (e.g., ethylene diamine), polyalkylene polyamines, polyepoxides, di- or polyglycidyl ethers and the like.

Suitable polyvalent metal cross-linking agents which can form ionic cross-linkages include oxides, hydroxides and weak acid salts (e.g., carbonate, acetate and the like) of alkaline earth metals (e.g., calcium magnesium) and zinc, including, for example, calcium oxide and zinc diacetate.

Of all of these types of cross-linking agents, the most preferred for use herein are diol derivatives and polyol derivatives, more specifically those selected from the group consisting of allyl sucrose, allyl pentaerythritol, divinyl glycol, divinyl polyethylene glycol and (meth)acrylic acid esters of diols.

In a preferred embodiment, the monomer composition from which the anionic polymer is derivable comprises the cross-linking agent in an amount of at most 1.0 mol-%, more preferably at most 0.1 mol-%, even more preferably at most about 0.01 mol-%, and most preferably at most 0.005 mol-% based on all monomers forming anionic polymer.

In a preferred embodiment, anionic polymer is a homopolymer of acrylic acid, optionally cross-linked, preferably with allyl sucrose or allyl pentaerythritol, in particular with allyl pentaerythritol. In another preferred embodiment, anionic polymer is a copolymer of acrylic acid and C₁₀-C₃₀-alkyl acrylate, optionally cross-linked, preferably with allyl pentaerythritol. In another preferred embodiment, anionic polymer is a so-called interpolymer, namely a homopolymer of acrylic acid, optionally cross-linked, preferably with allyl sucrose or allyl pentaerythritol; or a copolymer of acrylic acid and C₁₀-C₃₀-alkyl acrylate, optionally cross-linked, preferably with allyl pentaerythritol; which contain a block copolymer of polyethylene glycol and a long chain alkyl acid, preferably a C₈-C₃₀-alkyl acid. Polymers of this type are commercially available, e.g. under the trademark Carbopol®.

In another preferred embodiment, anionic polymer, preferably the pharmaceutical dosage form according to the invention does not contain a block copolymer of polyethylene glycol and an alkyl acid ester.

When anionic polymer is an interpolymer, it preferably has a viscosity in 1.0 wt.-% solution at pH 7.5 within the range of from 47,000 to 77,000 mPa·s, more preferably 52,000 to 72,000 mPa·s, still more preferably 57,000 to 67,000 mPa·s.

Preferably, at least some of the anionic functional groups contained in the anionic polymer are present in neutralized form, i.e. they are not present in their protonated forms, but are salts with salt-forming cations instead. Suitable salt-forming cations include alkali metal, ammonium, substituted ammonium and amines. More preferably, at least some of the anionic functional groups, e.g. carboxylate and/or sulfonate anions, are salts of sodium or potassium cations.

This percentage of neutralized anionic functional groups, based on the total amount of anionic functional groups, is referred to herein as the "degree of neutralization." In a preferred embodiment, the degree of neutralization is within the range of from 2.5±2.4%, more preferably 2.5±2.0%, still more preferably 2.5±1.5%, yet more preferably 2.5±1.0%, and most preferably 2.5±0.5%. In another preferred embodiment, the degree of neutralization is within the range of 35±30%, more preferably 35±25%, still more preferably 35±20%, yet more preferably 35±15%, most preferably 35±10%, and in particular 35±5%. In yet another preferred embodiment, the degree of neutralization is in the range of 65±30%, more preferably 65±25%, still more preferably 65±20%, yet more preferably 65±15%, most preferably 65±10%, and in particular 65±5%.

The content of anionic polymer ranges preferably from 0.1 wt.-% to 95 wt.-%, more preferably from 0.5 wt.-% to 80 wt.-%, still more preferably from 1.0 wt.-% to 50 wt.-%, and most preferably from 1.5 wt.-% to 20% wt.-%, and in particular 2.0 wt.-% to 10 wt.-%, based on the total weight of the pharmaceutical dosage form.

In a preferred embodiment, the content of anionic polymer amounts to 0.5 to 25 wt.-%, more preferably 1.0 to 20 wt.-%, still more preferably 2.0 to 22.5 wt.-%, yet more preferably 3.0 to 20 wt.-% and most preferably 4.0 to 17.5 wt.-% and in particular 5.0 to 15 wt.-%, based on the total weight of the pharmaceutical dosage form.

In a preferred embodiment, the content of anionic polymer is within the range of 5.0±4.5 wt.-%, more preferably 5.0±4.0 wt.-%, still more preferably 5.0±3.5 wt.-%, yet more preferably 5.0±3.0 wt.-%, most preferably 5.0±2.5 wt.-%, and in particular 5.0±2.0 wt.-%, based on the total weight of the pharmaceutical dosage form.

In another preferred embodiment, the content of anionic polymer is within the range of 10±9 wt.-%, more preferably 10±8 wt.-%, still more preferably 10±7 wt.-%, yet more preferably 10±6 wt.-%, most preferably 10±5 wt.-%, and in particular 10±2.5 wt.-%, based on the total weight of the pharmaceutical dosage form.

In still another preferred embodiment, the content of anionic polymer is within the range of 15±14 wt.-%, more preferably 15±12.5 wt.-%, still more preferably 15±10 wt.-%, yet more preferably 15±7.5 wt.-%, most preferably 15±5 wt.-%, and in particular 15±2.5 wt.-%, based on the total weight of the pharmaceutical dosage form.

In yet another preferred embodiment, the content of anionic polymer is within the range of 20±15 wt.-%, more preferably 20±12.5 wt.-%, still more preferably 20±10 wt.-%, yet more preferably 20±7.5 wt.-%, most preferably 20±5 wt.-%, and in particular 20±2.5 wt.-%, based on the total weight of the pharmaceutical dosage form.

In a preferred embodiment, the anionic polymer has a weight average molecular weight (Mw) of at least 100,000 g/mol, preferably at least 200,000 g/mol or at least 400,000 g/mol, more preferably in the range of about 500,000 g/mol to about 5,000,000 g/mol, and most preferably in the range of about 600,000 g/mol to about 2,000,000 g/mol. Suitable methods to determine Mw are known to a person skilled in the art. For instance, Mw can be determined by gel permeation chromatography (GPC).

In a preferred embodiment, the pK_{A} of the anionic polymer is 6.0±2.0, more preferably 6.0±1.5, even more preferably 6.0±1.0, and most preferably 6.0±0.5. In another preferred embodiment, the pK_{A} of the anionic polymer is 7.0±2.0, more preferably 7.0±1.5, even more preferably 7.0±1.0, and most preferably 7.0±0.5. In still another preferred embodiment, the pK_{A} of the anionic polymer is 8.0±2.0, more preferably 8.0±1.5, even more preferably 8.0±1.0, and most preferably 8.0±0.5.

In a preferred embodiment, the pH (in 1 wt% aqueous dispersion) of the anionic polymer is 3.0±3.0, more preferably 3.0±2.0, even more preferably 3.0±1.5, and most preferably 3.0±1.0.

In another preferred embodiment, the pH (in 1 wt% aqueous dispersion) of the anionic polymer is 6.0±3.0, more preferably 6.0±2.0, even more preferably 6.0±1.5, and most preferably 6.0±1.0.

The anionic polymer preferably exhibits a viscosity of 2,000 to 100,000 mPa•s (cp), more preferably 3,000 to 80,000 mPa•s, still more preferably 4,000 to 60,000 mPa•s, and in particular 4,000 to 11,000 mPa•s measured by means of a Brookfield viscometer (RVF, 20 rpm) in a 0.5 wt.-% aqueous solution at pH 7.5 and 25°C.

In a preferred embodiment, the anionic polymer exhibits a viscosity of more than 10,000 mPa•s (cp), preferably at least 11,000 mPa•s, more preferably at least 15,000 mPa•s, still more preferably at least 20,000 mPa•s or at least 30,000 mPa•s, measured by means of a Brookfield viscometer (RVF, 20 rpm) in a 0.5 wt.-% aqueous solution at pH 7.5 and 25°C.

In a preferred embodiment the relative weight ratio of said polyalkylene oxide and said anionic polymer is within the range of from 20:1 to 1:20, more preferably 18:1 to 1:10, still more preferably 16:1 to 1:5, yet more preferably 14:1 to 1:1, most preferably 12:1 to 2:1 and in particular 10:1 to 3:1. In a preferred embodiment, the relative weight ratio of said polyalkylene oxide and said anionic polymer is within the range of from 15:1 to 7:1, more preferably 10:1 to 6:1, most preferably 9:1 to 7:1.

Preferably, the content of said anionic polymer amounts to 0.5 to 25 wt.-%, more preferably 1.0 to 20 wt.-%, still more preferably 1.5 to 22.5 wt.-%, yet more preferably 2.0 to 20 wt.-% and most preferably 2.5 to 17.5 wt.-% and in particular 3.0 to 15 wt.-%, based on the total weight of the pharmaceutical dosage form.

In a preferred embodiment, the prolonged release matrix comprises an additional matrix polymer.

In a preferred embodiment according to the invention, the polyalkylene oxide having a weight average molecular weight of at least 200,000 g/mol and the anionic polymer are further combined with at least one additional polymer, preferably but not necessarily having a weight average molecular weight (Mw) of at least 200,000 g/mol, selected from the group consisting of polyethylene, polypropylene, polyvinyl chloride, polycarbonate, polystyrene, poly(hydroxy fatty acids), polycaprolactone, polyvinyl alcohol, polyesteramide, polyethylene succinate, polylactone, polyglycolide, polyurethane, polyvinylpyrrolidone, polyamide, polylactide, polylactide/glycolide, polylactone, polyglycolide, polyorthoester, polyanhydride, block polymers of polyethylene glycol and polybutylene terephthalate, polyanhydride, polyacetal, cellulose esters, cellulose ethers and copolymers thereof. Cellulose esters and cellulose ethers are particularly preferred, e.g. methylcellulose, ethylcellulose, hydroxymethylcellulose, hydroxyethylcellulose, hydroxypropylcellulose hydroxypropylmethylcellulose, carboxymethylcellulose, and the like.

In a preferred embodiment, said additional polymer is neither a polyalkylene oxide nor a polyalkylene glycol nor an anionic polymer. Nonetheless, the pharmaceutical dosage form may contain polyalkylene glycol, e.g. as plasticizer, but then, the pharmaceutical dosage form preferably is a quaternary mixture of polymers: polyalkylene oxide + anionic polymer + additional polymer + plasticizer.

In a particularly preferred embodiment, said additional polymer is a hydrophilic cellulose ester or cellulose ether, preferably hydroxypropylmethylcellulose (HPMC), hydroxypropylcellulose (HPC) or hydroxyethylcellulose (HEC), preferably having an average viscosity (preferably measured by capillary viscosimetry or rotational viscosimetry) of 1,000 to 150,000 mPas, more preferably 3,000 to 150,000. In a preferred embodiment, the average viscosity is within the range of 110,000±50,000 mPas, more preferably 110,000±40,000 mPas, still more preferably 110,000±30,000 mPas, most preferably 110,000±20,000 mPas, and in particular 100,000±10,000 mPas.

In a preferred embodiment the relative weight ratio of said polyalkylene oxide and said additional polymer is within the range of from 20:1 to 1:20, more preferably 15:1 to 1:10, still more preferably 10:1 to 1:5, yet more preferably 8:1 to 1:1, most preferably 8:1 to 2:1 and in particular 8:1 to 3:1. In a preferred embodiment, the relative weight ratio of said polyalkylene oxide and said additional polymer is within the range of from 10:1 to 2:1, more preferably 6:1 to 2:1, most preferably 3:1 to 2:1.

Preferably, the content of said additional polymer amounts to 0.5 to 25 wt.-%, more preferably 1.0 to 20 wt.-%, still more preferably 2.0 to 22.5 wt.-%, yet more preferably 3.0 to 20 wt.-% and most preferably 4.0 to 17.5 wt.-% and in particular 5.0 to 15 wt.-%, based on the total weight of the pharmaceutical dosage form.

In a preferred embodiment, the additional polymer is a cellulose ester or cellulose ether, preferably HPMC, having a content within the range of 10±8 wt.-%, more preferably 10±6 wt.-%, still more preferably 10±5 wt.-%, yet more preferably 10±4 wt.-%, most preferably 10±3 wt.-%, and in particular 10±2 wt.-%, based on the total weight of the pharmaceutical dosage form.

In another preferred embodiment, the additional polymer is a cellulose ester or cellulose ether, preferably HPMC, having a content within the range of 15±8 wt.-%, more preferably 15±6 wt.-%, still more preferably 15±5 wt.-%, yet more preferably 15±4 wt.-%, most preferably 15±3 wt.-%, and in particular 15±2 wt.-%, based on the total weight of the pharmaceutical dosage form.

All polymers are preferably employed as powders. They can be soluble in water.

Preferably, the pharmaceutical dosage form according to the invention is thermoformed, more preferably hot-melt extruded, although also other methods of thermoforming may be used in order to manufacture the pharmaceutical dosage form according to the invention, such as press-molding at elevated temperature or heating of tablets that were manufactured by conventional compression in a first step and then heated above the softening temperature of the polymer in the tablet in a second step to form hard tablets. In this regards, thermoforming means forming or molding of a mass after the application of heat. In a preferred embodiment, the pharmaceutical dosage form is thermoformed by hot-melt extrusion.

In a preferred embodiment, the pharmaceutical dosage form according to the invention has an overall density within the range of 1.19±0.30 g/cm3, more preferably 1.19±0.25 g/cm3, still more preferably 1.19±0.20 g/cm3, yet more preferably 1.19±0.15 g/cm3, most preferably 1.19±0.10 g/cm3, and in particular 1.19±0.05 g/cm3. Preferably, the overall density of the pharmaceutical dosage form according to the invention is 1.17±0.02 g/cm3, 1.19±0.02 g/cm3 or 1.21±0.02 g/cm3. Methods for measuring the density of a pharmaceutical dosage form are known to a person skilled in the art. The overall density of a pharmaceutical dosage form can for example be determined by means of the mercury porosimetry method or the helium pycnometer method as described in Ph. Eur.

In a preferred embodiment, the pharmaceutical dosage form has a total weight within the range of 100±75 mg, more preferably 100±50 mg, most preferably 100±25 mg. In another preferred embodiment, the pharmaceutical dosage form has a total weight within the range of 200±75 mg, more preferably 200±50 mg, most preferably 200±25 mg. In another preferred embodiment, the pharmaceutical dosage form has a total weight within the range of 250±75 mg, more preferably 250±50 mg, most preferably 250±25 mg. In still another preferred embodiment, the pharmaceutical dosage form has a total weight within the range of 300±75 mg, more preferably 300±50 mg, most preferably 300±25 mg. In yet another preferred embodiment, the pharmaceutical dosage form has a total weight within the range of 400±75 mg, more preferably 400±50 mg, most preferably 400±25 mg.

In a preferred embodiment, the pharmaceutical dosage form has a total weight within the range of 500±250 mg, more preferably 500±200 mg, most preferably 500±150 mg. In another preferred embodiment, the pharmaceutical dosage form has a total weight within the range of 750±250 mg, more preferably 750±200 mg, most preferably 750±150 mg. In another preferred embodiment, the pharmaceutical dosage form has a total weight within the range of 1000±250 mg, more preferably 1000±200 mg, most preferably 1000±150 mg. In still another preferred embodiment, the pharmaceutical dosage form has a total weight within the range of 1250±250 mg, more preferably 1250±200 mg, most preferably 1250±150 mg.

The pharmaceutical dosage form according to the invention contains, as pharmacologically active ingredient, being an opioid agonist, preferably oxymorphone or oxycodone. For the purpose of the specification, the term pharmacologically active ingredient, being an opioid agonist also includes the free base and the physiologically acceptable salts thereof.

According to the ATC index, opioid agonists (opioids) are divided into natural opium alkaloids, phenylpiperidine derivatives, diphenylpropylamine derivatives, benzomorphan derivatives, oripavine derivatives, morphinan derivatives and others. Examples of natural opium alkaloids are morphine, opium, hydromorphone, nicomorphine, oxycodone, dihydrocodeine, diamorphine, papaveretum, and codeine. Further opioid agonists are, for example, ethylmorphine, hydrocodone, oxymorphone, and the physiologically acceptable derivatives thereof or compounds, preferably the salts and solvates thereof, preferably the hydrochlorides thereof, physiologically acceptable enantiomers, stereoisomers, diastereomers and racemates and the physiologically acceptable derivatives thereof, preferably ethers, esters or amides.

Further preferred opioid agonists include N-(1-methyl-2-piperidinoethyl)-N-(2-pyridyl)propion-amide, (1R,2R)-3-(3-dimethylamino-1-ethyl-2-methyl-propyl)phenol (tapentadol), (1R,2R,4S)-2-(dimethylamino)methyl-4-(p-fluorobenzyloxy)-1-(m-methoxyphenyl)cyclohexanol, (1R,2R)-3-(2-dimethylaminomethyl-cyclohexyl)phenol, (1S,2S)-3-(3-dimethylamino-1-ethyl-2-methylpropyl)phenol, (2R,3R)-1-dimethylamino-3(3-methoxyphenyl)-2-methyl-pentan-3-ol, (1RS, 3RS,6RS)-6-dimethylaminomethyl-1-(3-methoxyphenyl)-cyclohexane-1,3-diol, preferably as racemate, 3-(2-dimethylaminomethyl-1-hydroxy-cyclohexyl)phenyl 2-(4-isobutyl-phenyl)-propionate, 3-(2-dimethylam inomethyl-1-hydroxy-cyclohexyl)phenyl 2-(6-methoxy-naphthalen-2-yl)propionate, 3-(2-dimethylaminomethyl-cyclohex-1-enyl)-phenyl 2-(4-isobutyl-phenyl)-propionate, 3-(2-dimethylaminomethyl-cyclohex-1-enyl)-phenyl 2-(6-methoxy-naphthalen-2-yl)propionate, (RR-SS)-2-acetoxy-4-trifluoromethyl-benzoic acid 3-(2-dimethylaminomethyl-1-hydroxy-cyclohexyl)-phenyl ester, (RR-SS)-2-hydroxy-4-trifluoromethyl-benzoic acid 3-(2-dimethylaminomethyl-1-hydroxy-cyclohexyl)-phenyl ester, (RR-SS)-4-chloro-2-hydroxy-benzoic acid 3-(2-dimethylaminomethyl-1-hydroxy-cyclohexyl)-phenyl ester, (RR-SS)-2-hydroxy-4-methyl-benzoic acid 3-(2-dimethylaminomethyl-1-hydroxy-cyclohexyl)-phenyl ester, (RR-SS)-2-hydroxy-4-methoxy-benzoic acid 3-(2-dimethylaminomethyl-1-hydroxy-cyclohexyl)-phenyl ester, (RR-SS)-2-hydroxy-5-nitro-benzoic acid 3-(2-dimethylaminomethyl-1-hydroxycyclohexyl)-phenyl ester, (RR-SS)-2',4'-difluoro-3-hydroxy-biphenyl-4-carboxylic acid 3-(2-dimethylaminomethyl-1-hydroxy-cyclohexyl)-phenyl ester, 1,1-(3-dimethylamino-3-phenyl-pentamethylen)-6-fluor-1,3,4,9-tetrahydropyrano[3,4-b]indole, in particular its hemicitrate; 1,1-[3-dimethylamino-3-(2-thienyl)pentamethylen]-1,3,4,9-tetrahydropyrano[3,4-b]indole, in particular its citrate; and 1,1-[3-dimethylamino-3-(2-thienyl)pentamethylen]-1,3,4,9-tetra-hydropyrano[3,4-b]-6-fluoro-indole, in particular its hemicitrate, and corresponding stereoisomeric compounds, in each case the corresponding derivatives thereof, physiologically acceptable enantiomers, stereoisomers, diastereomers and racemates and the physiologically acceptable derivatives thereof, e.g. ethers, esters or amides, and in each case the physiologically acceptable compounds thereof, in particular the salts thereof and solvates, e.g. hydrochlorides.

Particularly preferred opioid agonists include oxymorphone, oxycodone, hydromorphone, and the physiologically acceptable salts thereof. In a particularly preferred embodiment, the opioid agonist is oxycodone or a physiologically acceptable salt thereof.

In another preferred embodiment, the pharmacologically active ingredient is selected from the group consisting of acetaminophen, albuterol, alendronate, alfuzosin, alprazolam, ambrisentan, amoxicillin, amphetamine salts, aspirin, atomoxetine, benzonatate,bisacodyl, bosentan, brompheniramine, budesonide, bupropion, carbamazepine, cefuroxime, chloral hydrate, cinacalcet, ciprofloxacin, ciprofloxicin, clarithromycin, clonidine, colestipol, cyclobenzaprine, cyclophosphamide, dabigatran, dalfampridine, darifenacin, dasatinib, dexlansoprazole, dexmethylphenidate, diclofenac, didanosine, diltiazem, disopyramide, divalproex, docusate, donepezil, doxazosin, doxycycline, duloxetine, dutasteride, dutasteride/tamsulosin, ergocalciferol, ergotamine, erythromycin, esomeprazole, etravirine, everolismus, felodipine, fentanyl, ferrous gluconate, ferrous sulfate, fesoterodine, finasteride, fluoxetine, fluvastatin, fluvoxamine, gabapentin enacarbil, gabapentin, galantamine, ganciclovir, glipizide, guaifenesin, guanfacine, hydromorphone, hydroxyurea, hyoscyamine, ibandronate, ibuprofen, imatinib,indinavir, indomethacin, isosorbide, isotretinoin, isradipine, lamotrigine, lansoprazole, lenadilomide, levetiracetam, levodopa/carbidopa, lithium, lovastatin, lubiprostone, memantine, mesalamine, metformin, methylphenidate, metoprolol succinate, metronidazole, minocycline, morphine sulfate, morphine, mycophenolate, naproxen, naproxen/esomeprazole, nevirapine, nicardipine, nicotine, nicotinic acid, nifedipine, nilotinib, nisoldipine, nitroglycerin, omeprazole, omeprazole/sodium bicarbonate, orphenadrine citrate, oxybutynin, oxycodone, pabcreaslipase, paliperidone, pancrelipase, pantoprazole, paroxetine, pazopanib, pentoxifylline, piroxicam, potassium bicarbonate, potassium chloride, potassium citrate, potassium, praziquantel, propafenone, propanolol, pyridosigmine, quetiapine, rabeprazole, raloxifene, ranolazine, risedronate, ritonavir, ropinirole, sevelamer carbonate, sevelamer, sirolismus, solifenacin, sulfasalazine, tamsulosin, tapentadol, telithromycin, temozolomide, theophylline, tipranavir, tolterodine, topiramate, tramadol, trazodone, valganciclovir, venlafaxine, verapamil, vorinostat, zileutonzolpidem, and the physiologically acceptable salts thereof.

The content of the pharmacologically active ingredient, being an opioid agonist in the pharmaceutical dosage form is not limited.

Preferably, the content of the pharmacologically active ingredient, being an opioid agonist is within the range of from 0.01 to 80 wt.-%, more preferably 0.1 to 50 wt.-%, still more preferably 1 to 25 wt.-%, based on the total weight of the pharmaceutical dosage form. In a preferred embodiment, the content of pharmacologically active ingredient, being an opioid agonist is within the range of from 7±6 wt.-%, more preferably 7±5 wt.-%, still more preferably 5±4 wt.-%, 7±4 wt.-% or 9±4 wt.-%, most preferably 5±3 wt.-%, 7±3 wt.-% or 9±3 wt.-%, and in particular 5±2 wt.-%, 7±2 wt.-% or 9±2 wt.-%, based on the total weight of the pharmaceutical dosage form. In another preferred embodiment, the content of pharmacologically active ingredient, being an opioid agonist is within the range of from 11±10 wt.-%, more preferably 11±9 wt.-%, still more preferably 9±6 wt.-%, 11±6 wt.-%, 13±6 wt.-% or 15±6 wt.-%, most preferably 11±4 wt.-%, 13±4 wt.-% or 15±4 wt.-%, and in particular 11±2 wt.-%, 13±2 wt.-% or 15±2 wt.-%, based on the total weight of the pharmaceutical dosage form. In a further preferred embodiment, the content of pharmacologically active ingredient, being an opioid agonist is within the range of from 20±6 wt.-%, more preferably 20±5 wt.-%, still more preferably 20±4 wt.-%, most preferably 20±3 wt.-%, and in particular 20±2 wt.-%, based on the total weight of the pharmaceutical dosage form.

Preferably, the total amount of the pharmacologically active ingredient, being an opioid agonist that is contained in the pharmaceutical dosage form is within the range of from 0.01 to 200 mg, more preferably 0.1 to 190 mg, still more preferably 1.0 to 180 mg, yet more preferably 1.5 to 160 mg, most preferably 2.0 to 100 mg and in particular 2.5 to 80 mg.

In a preferred embodiment, the pharmacologically active ingredient, being an opioid agonist is contained in the pharmaceutical dosage form in an amount of 7.5±5 mg, 10±5 mg, 20±5 mg, 30±5 mg, 40±5 mg, 50±5 mg, 60±5 mg, 70±5 mg, 80±5 mg, 90±5 mg, 100±5 mg, 110±5 mg, 120±5 mg, 130±5, 140±5 mg, 150±5 mg, or 160±5 mg. In another preferred embodiment, the pharmacologically active ingredient, being an opioid agonist is contained in the pharmaceutical dosage form in an amount of 5±2.5 mg, 7.5±2.5 mg, 10±2.5 mg, 15±2.5 mg, 20±2.5 mg, 25±2.5 mg, 30±2.5 mg, 35±2.5 mg, 40±2.5 mg, 45±2.5 mg, 50±2.5 mg, 55±2.5 mg, 60±2.5 mg, 65±2.5 mg, 70±2.5 mg, 75±2.5 mg, 80±2.5 mg, 85±2.5 mg, 90±2.5 mg, 95±2.5 mg, 100±2.5 mg, 105±2.5 mg, 110±2.5 mg, 115±2.5 mg, 120±2.5 mg, 125±2.5 mg, 130±2.5 mg, 135±2.5 mg, 140±2.5 mg, 145±2.5 mg, 150±2.5 mg, 155±2.5 mg, or 160±2.5 mg.

In a preferred embodiment, opioid agonist is oxymorphone, preferably its HCI salt, and the pharmaceutical dosage form is adapted for administration twice daily. In this embodiment, opioid agonist is preferably contained in the pharmaceutical dosage form in an amount of from 5 to 60 mg. In another particularly preferred embodiment, the opioid agonist is oxymorphone, preferably its HCI salt, and the pharmaceutical dosage form is adapted for administration once daily. In this embodiment, opioid agonist is preferably contained in the pharmaceutical dosage form in an amount of from 10 to 100 mg.

In another preferred embodiment, opioid agonist is oxycodone, preferably its HCI salt, and the pharmaceutical dosage form is adapted for administration twice daily. In this embodiment, opioid agonist is preferably contained in the pharmaceutical dosage form in an amount of from 5 to 80 mg, preferably 5 mg, 10 mg, 20 mg or 40 mg. In another particularly preferred embodiment, the opioid agonist is oxycodone, preferably its HCI salt, and the pharmaceutical dosage form is adapted for administration once daily. In this embodiment, opioid agonist is preferably contained in the pharmaceutical dosage form in an amount of from 10 to 320 mg.

In still another particularly preferred embodiment, opioid agonist is hydromorphone, preferably its HCl, and the pharmaceutical dosage form is adapted for administration twice daily. In this embodiment, opioid agonist is preferably contained in the pharmaceutical dosage form in an amount of from 2 to 52 mg. In another particularly preferred embodiment, opioid agonist is hydromorphone, preferably its HCI salt, and the pharmaceutical dosage form is adapted for administration once daily. In this embodiment, opioid agonist is preferably contained in the pharmaceutical dosage form in an amount of from 4 to 104 mg.

The pharmaceutical dosage form according to the invention is characterized by excellent storage stability. Preferably, the pharmaceutical dosage form according to the invention has a storage stability at 40°C for at least 3 months, more preferably at least 4 months, still more preferably at least 5 months, yet more preferably at least 6 months, even more preferably at least 7 months, most preferably at least 8 months, and in particular at least 9 months. Storage stability is preferably determined in accordance with the EMEA Guideline CPMP/ICH/2736/99-ICH Q1A (R2), preferably in the version valid for 2012, and/or the pharmaceutical dosage form according to the invention preferably fulfills the impurity limits given by the API monographs Ph. Eur. (7th edition, 2011) after 3 months of storage at 40°C and 75% RH.

Preferably, after storage for 4 weeks at 40°C and 75% rel. humidity, the content of pharmacologically active ingredient, being an opioid agonist and opioid antagonist in each case amounts to at least 90%, more preferably at least 91%, still more preferably at least 92%, yet more preferably at least 93%, most preferably at least 94% and in particular at least 95%, of its original content before storage. Suitable methods for measuring the content of the pharmacologically active ingredient, being an opioid agonist and opioid antagonist in the pharmaceutical dosage form are known to the skilled artisan. In this regard it is referred to the Eur. Ph. or the USP, especially to reversed phase HPLC analysis. Preferably, the pharmaceutical dosage form is stored in closed, preferably sealed containers, most preferably being equipped with an oxygen scavenger, in particular with an oxygen scavenger that is effective even at low relative humidity.

In a preferred embodiment, after oral administration of the pharmaceutical dosage form according to the invention, in vivo the average peak plasma level (Cₘₐₓ) of the pharmacologically active ingredient, being an opioid agonist is on average reached after tₘₐₓ 3.0±2.5 h, more preferably after tₘₐₓ 3.0±2.0 h, still more preferably after tₘₐₓ 3.0±1.5 h, most preferably after tₘₐₓ 3.0±1.0 h and in particular after tₘₐₓ 3.0±0.5 h. In a preferred embodiment, after oral administration of the pharmaceutical dosage form according to the invention, in vivo the average peak plasma level (Cₘₐₓ) of the pharmacologically active ingredient, being an opioid agonist is on average reached after tₘₐₓ 4.0±2.5 h, more preferably after tₘₐₓ 4.0±2.0 h, still more preferably after tₘₐₓ 4.0±1.5 h, most preferably after tₘₐₓ 4.0±1.0 h and in particular after tₘₐₓ 4.0±0.5 h. In another preferred embodiment, after oral administration of the pharmaceutical dosage form according to the invention, in vivo the average peak plasma level (Cₘₐₓ) of the pharmacologically active ingredient, being an opioid agonist is on average reached after tₘₐₓ 5.0±2.5 h, more preferably after tₘₐₓ 5.0±2.0 h, still more preferably after tₘₐₓ 5.0±1.5 h, most preferably after tₘₐₓ 5.0±1.0 h and in particular after tₘₐₓ 5.0±0.5 h. In still another preferred embodiment, after oral administration of the pharmaceutical dosage form according to the invention, in vivo the average peak plasma level (Cₘₐₓ) of the pharmacologically active ingredient, being an opioid agonist is on average reached after tₘₐₓ 6.0±2.5 h, more preferably after tₘₐₓ 6.0±2.0 h, still more preferably after tₘₐₓ 6.0±1.5 h, most preferably after tₘₐₓ 6.0±1.0 h and in particular after tₘₐₓ 6.0±0.5 h.

In a preferred embodiment, the average value for t_{1/2} of the pharmacologically active ingredient, being an opioid agonist after oral administration of the pharmaceutical dosage form according to the invention in vivo is 3.0±2.5 h, more preferably 3.0±2.0 h, still more preferably 3.0±1.5 h, most preferably 3.0±1.0 h, and in particular 3.0±0.5 h. In a preferred embodiment, the average value for t_{1/2} of the pharmacologically active ingredient, being an opioid agonist after oral administration of the pharmaceutical dosage form according to the invention in vivo is 4.0±2.5 h, more preferably 4.0±2.0 h, still more preferably 4.0±1.5 h, most preferably 4.0±1.0 h, and in particular 4.0±0.5 h. In another preferred embodiment, the average value for t_{1/2} of the pharmacologically active ingredient, being an opioid agonist after oral administration of the pharmaceutical dosage form according to the invention in vivo is preferably 5.0±2.5 h, more preferably 5.0±2.0 h, still more preferably 5.0±1.5 h, most preferably 5.0±1.0 h, and in particular 5.0±0.5 h. In still another preferred embodiment, the average value for t_{1/2} of the pharmacologically active ingredient, being an opioid agonist after oral administration of the pharmaceutical dosage form according to the invention in vivo is preferably 6.0±2.5 h, more preferably 6.0±2.0 h, still more preferably 6.0±1.5 h, most preferably 6.0±1.0 h, and in particular 6.0±0.5 h.

Preferably, Cₘₐₓ of the pharmacologically active ingredient, being an opioid agonist does not exceed 0.01 ng/ml, or 0.05 ng/ml, or 0.1 ng/ml, or 0.5 ng/ml, or 1.0 ng/ml, or 2.5 ng/ml, or 5 ng/ml, or 10 ng/ml, or 20 ng/ml, or 30 ng/ml, or 40 ng/ml, or 50 ng/ml, or 75 ng/ml, or 100 ng/ml, or 150 ng/ml, or 200 ng/ml, or 250 ng/ml, or 300 ng/ml, or 350 ng/ml, or 400 ng/ml, or 450 ng/ml, or 500 ng/ml, or 750 ng/ml, or 1000 ng/ml.

The opioid antagonist is selected from the group consisting of naltrexone, naloxone, nalmefene, cyclazacine, levallorphan, , pharmaceutically acceptable salts thereof and mixtures thereof.

Opioid antagonists that are not or only poorly bioavailable upon oral administration, but much better bioavailable upon parenteral administration, are particularly preferred.

Opioid antagonists suitable for a given opioid agonist are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains an opioid antagonist selected from the group consisting of naloxone, naltrexone, nalmefene, nalide, nalmexone, nalorphine or naluphine, in each case optionally in the form of a corresponding physiologically acceptable compound, in particular in the form of a base, a salt or solvate.

Naloxone and nalmexone as well as their physiologically acceptable salts are preferred opioid antagonists.

Naloxone is particularly preferred as opioid antagonist, preferably its hydrochloride, more preferably the dihydrate of the hydrochloride.

The content of the opioid antagonist in the pharmaceutical dosage form is not limited.

Preferably, the content of the opioid antagonist in the pharmaceutical dosage form according to the invention is such that it is at least sufficient to locally block the opioid receptors in the intestine thereby suppressing obstipation that would otherwise be induced by the opioid agonist. Preferably, however, the content of the opioid antagonist is increased to an amount sufficient to counter the effect of the opioid agonist when the pharmaceutical dosage form is tampered with, particularly by liquid extraction of the active ingredients and parenteral administration of the liquid extract. There is indication that the quantity needed for this effect is higher than the quantity needed for suppression of obstipation.

Preferably, the content of the opioid antagonist is within the range of from 0.01 to 80 wt.-%, more preferably 0.1 to 50 wt.-%, still more preferably 1 to 25 wt.-%, based on the total weight of the pharmaceutical dosage form. In a preferred embodiment, the content of opioid antagonist is within the range of from 7±6 wt.-%, more preferably 7±5 wt.-%, still more preferably 5±4 wt.-%, 7±4 wt.-% or 9±4 wt.-%, most preferably 5±3 wt.-%, 7±3 wt.-% or 9±3 wt.-%, and in particular 5±2 wt.-%, 7±2 wt.-% or 9±2 wt.-%, based on the total weight of the pharmaceutical dosage form. In another preferred embodiment, the content of opioid antagonist is within the range of from 11±10 wt.-%, more preferably 11±9 wt.-%, still more preferably 9±6 wt.-%, 11±6 wt.-%, 13±6 wt.-% or 15±6 wt.-%, most preferably 11±4 wt.-%, 13±4 wt.-% or 15±4 wt.-%, and in particular 11±2 wt.-%, 13±2 wt.-% or 15±2 wt.-%, based on the total weight of the pharmaceutical dosage form. In a further preferred embodiment, the content of opioid antagonist is within the range of from 20±6 wt.-%, more preferably 20±5 wt.-%, still more preferably 20±4 wt.-%, most preferably 20±3 wt.-%, and in particular 20±2 wt.-%, based on the total weight of the pharmaceutical dosage form.

Preferably, the total amount of the opioid antagonist that is contained in the pharmaceutical dosage form is within the range of from 0.01 to 200 mg, more preferably 0.1 to 190 mg, still more preferably 1.0 to 180 mg, yet more preferably 1.5 to 160 mg, most preferably 2.0 to 100 mg and in particular 2.5 to 80 mg.

In a preferred embodiment, the opioid antagonist is contained in the pharmaceutical dosage form in an amount of 1.0±0.5 mg, 2.0±1.0 mg, 3.0±1.0 mg, 4.0±1.0 mg, 5.0±1.0 mg, 7.5±5 mg, 10±5 mg, 20±5 mg, 30±5 mg, 40±5 mg, 50±5 mg, 60±5 mg, 70±5 mg, 80±5 mg, 90±5 mg, 100±5 mg, 110±5 mg, 120±5 mg, 130±5, 140±5 mg, 150±5 mg, or 160±5 mg. In another preferred embodiment, the opioid antagonist is contained in the pharmaceutical dosage form in an amount of 5±2.5 mg, 7.5±2.5 mg, 10±2.5 mg, 15±2.5 mg, 20±2.5 mg, 25±2.5 mg, 30±2.5 mg, 35±2.5 mg, 40±2.5 mg, 45±2.5 mg, 50±2.5 mg, 55±2.5 mg, 60±2.5 mg, 65±2.5 mg, 70±2.5 mg, 75±2.5 mg, 80±2.5 mg, 85±2.5 mg, 90±2.5 mg, 95±2.5 mg, 100±2.5 mg, 105±2.5 mg, 110±2.5 mg, 115±2.5 mg, 120±2.5 mg, 125±2.5 mg, 130±2.5 mg, 135±2.5 mg, 140±2.5 mg, 145±2.5 mg, 150±2.5 mg, 155±2.5 mg, or 160±2.5 mg.

Preferably, the relative weight ratio of the opioid agonist and the opioid antagonist is within the range of from 20:1 to 1:5, more preferably 15:1 to 1:4, still more preferably 10:1 to 1:3, yet more preferably 5:1 to 1:2, even more preferably 3.5:1 to 1:1.5, most preferably 3:1 to 1:1, and in particular 2.5:1 to 1.5:1.

The purpose of the opioid antagonist that is contained in the pharmaceutical dosage form according to the invention is on the one hand associated with the tamper resistance of the pharmaceutical dosage form, especially when the pharmaceutical dosage form is administered by a non-prescribed route of administration, particularly intravenous administration of a liquid extract. Under these circumstances, the opioid antagonist preferably evolves its antagonizing effect thereby avoiding misuse of the opioid agonist. On the other hand, the purpose of the opioid antagonist is preferably to reduce undesired adverse events, particularly to counter obstipation that would be otherwise induced by the opioid agonist. This is achieved by locally blocking the pharmacological effect of the opioid agonist at the opioid receptors in the intestine upon prescribed oral administration of the pharmaceutical dosage form.

In a particularly preferred embodiment, the opioid antagonist is naloxone, preferably its HCI salt, and the pharmaceutical dosage form is adapted for administration twice daily. In this embodiment, the opioid antagonist is preferably contained in the pharmaceutical dosage form in an amount of from 1.0 to 40 mg.

In a particularly preferred embodiment, the opioid agonist is oxycodone, preferably its hydrochloride, and the opioid antagonist is naloxone, preferably its hydrochloride. Preferred contents A1 to A24 of said opioid agonist and said opioid antagonist for this embodiment are summarized in the table here below:

| mg | A1 | A2 | A3 | A4 | A5 | A6 |
|---|---|---|---|---|---|---|
| opioid agonist | 5.0±2.0 | 10±2.0 | 15±2.0 | 20±2.0 | 25±2.0 | 30±2.0 |
| opioid antagonist | 2.5±2.0 | 5.0±4.5 | 7.5±7.0 | 10±9.5 | 12.5±12.0 | 15±14.5 |
| | | | | | | |

| mg | A7 | A8 | A9 | A10 | A11 | A12 |
|---|---|---|---|---|---|---|
| opioid agonist | 35±2.0 | 40±2.0 | 50±2.0 | 60±2.0 | 70±2.0 | 80±2.0 |
| opioid antagonist | 17.5±17.0 | 20±19.5 | 25±24.5 | 30±29.5 | 35±34.5 | 40±39.5 |
| | | | | | | |

| mg | A13 | A14 | A15 | A16 | A17 | A18 |
|---|---|---|---|---|---|---|
| opioid agonist | 5.0±2.0 | 10±2.0 | 15±2.0 | 20±2.0 | 25±2.0 | 30±2.0 |
| opioid antagonist | 2.5±2.0 | 5.0±2.0 | 7.5±2.0 | 10±2.0 | 12.5±2.0 | 15±2.0 |
| | | | | | | |

| mg | A19 | A20 | A21 | A22 | A23 | A24 |
|---|---|---|---|---|---|---|
| opioid agonist | 35±2.0 | 40±2.0 | 50±2.0 | 60±2.0 | 70±2.0 | 80±2.0 |
| opioid antagonist | 17.5±2.0 | 20±2.0 | 25±2.0 | 30±2.0 | 35±2.0 | 40±2.0 |

In a preferred embodiment, after oral administration of the pharmaceutical dosage form according to the invention, in vivo the average peak plasma level (Cₘₐₓ) of the opioid antagonist is on average reached after tₘₐₓ 3.0±2.5 h, more preferably after tₘₐₓ 3.0±2.0 h, still more preferably after tₘₐₓ 3.0±1.5 h, most preferably after tₘₐₓ 3.0±1.0 h and in particular after tₘₐₓ 3.0±0.5 h. In another preferred embodiment, after oral administration of the pharmaceutical dosage form according to the invention, in vivo the average peak plasma level (Cₘₐₓ) of the opioid antagonist is on average reached after tₘₐₓ 3.4±2.5 h, more preferably after tₘₐₓ 3.4±2.0 h, still more preferably after tₘₐₓ 3.4±1.5 h, most preferably after tₘₐₓ 3.4±1.0 h and in particular after tₘₐₓ 3.4±0.5 h. In still another preferred embodiment, after oral administration of the pharmaceutical dosage form according to the invention, in vivo the average peak plasma level (Cₘₐₓ) of the opioid antagonist is on average reached after tₘₐₓ 4.0±2.5 h, more preferably after tₘₐₓ 4.0±2.0 h, still more preferably after tₘₐₓ 4.0±1.5 h, most preferably after tₘₐₓ 4.0±1.0 h and in particular after tₘₐₓ 4.0±0.5 h. In yet another preferred embodiment, after oral administration of the pharmaceutical dosage form according to the invention, in vivo the average peak plasma level (Cₘₐₓ) of the opioid antagonist is on average reached after tₘₐₓ 5.0±2.5 h, more preferably after tₘₐₓ 5.0±2.0 h, still more preferably after tₘₐₓ 5.0±1.5 h, most preferably after tₘₐₓ 5.0±1.0 h and in particular after tₘₐₓ 5.0±0.5 h. In still another preferred embodiment, after oral administration of the pharmaceutical dosage form according to the invention, in vivo the average peak plasma level (Cₘₐₓ) of the opioid antagonist is on average reached after tₘₐₓ 6.0±2.5 h, more preferably after tₘₐₓ 6.0±2.0 h, still more preferably after tₘₐₓ 6.0±1.5 h, most preferably after tₘₐₓ 6.0±1.0 h and in particular after tₘₐₓ 6.0±0.5 h.

In a preferred embodiment, the average value for t_{1/2} of the opioid antagonist after oral administration of the pharmaceutical dosage form according to the invention in vivo is 4.0±2.5 h, more preferably 4.0±2.0 h, still more preferably 4.0±1.5 h, most preferably 4.0±1.0 h, and in particular 4.0±0.5 h. In another preferred embodiment, the average value for t_{1/2} of the opioid antagonist after oral administration of the pharmaceutical dosage form according to the invention in vivo is 4.3±2.5 h, more preferably 4.3±2.0 h, still more preferably 4.3±1.5 h, most preferably 4.3±1.0 h, and in particular 4.3±0.5 h. In still another preferred embodiment, the average value for t_{1/2} of the opioid antagonist after oral administration of the pharmaceutical dosage form according to the invention in vivo is preferably 5.0±2.5 h, more preferably 5.0±2.0 h, still more preferably 5.0±1.5 h, most preferably 5.0±1.0 h, and in particular 5.0±0.5 h. In yet another preferred embodiment, the average value for t_{1/2} of the opioid antagonist after oral administration of the pharmaceutical dosage form according to the invention in vivo is preferably 6.0±2.5 h, more preferably 6.0±2.0 h, still more preferably 6.0±1.5 h, most preferably 6.0±1.0 h, and in particular 6.0±0.5 h.

In a preferred embodiment, Cₘₐₓ of the opioid antagonist is below Cₘₐₓ of the opioid agonist. Preferably, Cₘₐₓ of the opioid antagonist is at most 90%, more preferably at most 80%, still more preferably at most 70%, yet more preferably at most 65%, even more preferably at most 60%, most preferably at most 55% and in particular at most 50% of Cₘₐₓ of the opioid agonist.

Preferably, Cₘₐₓ of the opioid antagonist does not exceed 0.01 ng/ml, or 0.05 ng/ml, or 0.1 ng/ml, or 0.5 ng/ml, or 1.0 ng/ml, or 2.5 ng/ml, or 5 ng/ml, or 10 ng/ml, or 20 ng/ml, or 30 ng/ml, or 40 ng/ml, or 50 ng/ml, or 75 ng/ml, or 100 ng/ml, or 150 ng/ml, or 200 ng/ml, or 250 ng/ml, or 300 ng/ml, or 350 ng/ml, or 400 ng/ml, or 450 ng/ml, or 500 ng/ml, or 750 ng/ml, or 1000 ng/ml.

Preferably, at any point in time during 8 h, more preferably 10 h, most preferably 12 h, after oral administration of the pharmaceutical dosage form, the plasma concentration of the opioid antagonist is below the plasma concentration of the opioid agonist. Preferably, at any point in time during 8 h, more preferably 10 h, most preferably 12 h, after oral administration of the pharmaceutical dosage form, the plasma concentration of the opioid antagonist is at most 90%, more preferably at most 80%, still more preferably at most 70%, yet more preferably at most 65%, even more preferably at most 60%, most preferably at most 55% and in particular at most 50% of the plasma concentration of the opioid agonist at the same point in time.

In a preferred embodiment, the pharmaceutical dosage form according to the invention contains an opioid antagonist but no substances which irritate the nasal passages and/or pharynx, i.e. substances which, when administered via the nasal passages and/or pharynx, bring about a physical reaction which is either so unpleasant for the patient that he/she does not wish to or cannot continue administration, for example burning, or physiologically counteracts taking of the corresponding active compound, for example due to increased nasal secretion or sneezing. Further examples of substances which irritate the nasal passages and/or pharynx are those which cause burning, itching, urge to sneeze, increased formation of secretions or a combination of at least two of these stimuli. Corresponding substances and the quantities thereof which are conventionally to be used are known to the person skilled in the art. Some of the substances which irritate the nasal passages and/or pharynx are accordingly based on one or more constituents or one or more plant parts of a hot substance drug. Corresponding hot substance drugs are known per se to the person skilled in the art and are described, for example, in "Pharmazeutische Biologie - Drogen und ihre Inhaltsstoffe" by Prof. Dr. Hildebert Wagner, 2nd., revised edition, Gustav Fischer Verlag, Stuttgart-New York, 1982, pages 82 et seq.. The corresponding description is hereby introduced as a reference and is deemed to be part of the disclosure.

The pharmaceutical dosage form according to the invention furthermore preferably contains an opioid antagonist but no emetic. Emetics are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no emetic based on one or more constituents of ipecacuanha (ipecac) root, for example based on the constituent emetine, as are, for example, described in "Pharmazeutische Biologie - Drogen und ihre Inhaltsstoffe" by Prof. Dr. Hildebert Wagner, 2nd, revised edition, Gustav Fischer Verlag, Stuttgart, New York, 1982. The corresponding literature description is hereby introduced as a reference and is deemed to be part of the disclosure. The pharmaceutical dosage form according to the invention preferably also contains no apomorphine as an emetic.

The pharmaceutical dosage form according to the invention preferably also contains an opioid antagonist but no bitter substance. Bitter substances and the quantities effective for use may be found in US-2003/0064099 A1, the corresponding disclosure of which should be deemed to be the disclosure of the present application and is hereby introduced as a reference. Examples of bitter substances are aromatic oils, such as peppermint oil, eucalyptus oil, bitter almond oil, menthol, fruit aroma substances, aroma substances from lemons, oranges, limes, grapefruit or mixtures thereof, and/or denatonium benzoate.

The pharmaceutical dosage form according to the invention accordingly preferably contains an opioid antagonist but no aversive agent, i.e. neither substances which irritate the nasal passages and/or pharynx, nor emetics, nor bitter substances.

Preferably, the pharmaceutical dosage form according to the invention contains no neuroleptics, for example a compound selected from the group consisting of haloperidol, promethacine, fluphenazine, perphenazine, levomepromazine, thioridazine, perazine, chlorpromazine, chlorprothixine, zuclopenthixol, flupentixol, prothipendyl, zotepine, benperidol, pipamperone, melperone and bromperidol.

Besides (i) the pharmacologically active ingredient, being an opioid agonist, (ii) the opioid antagonist, (iii) the polyalkylene oxide and (iv) the anionic polymer, the pharmaceutical dosage form according to the invention may contain further constituents, such as conventional pharmaceutical excipients.

Preferably, the pharmaceutical dosage form according to the invention contains a plasticizer.

The plasticizer improves the processability of the polyalkylene oxide. A preferred plasticizer is polyalkylene glycol, like polyethylene glycol, triacetin, fatty acids, fatty acid esters, waxes and/or microcrystalline waxes. Particularly preferred plasticizers are polyethylene glycols, such as PEG 6000.

Preferably, the content of the plasticizer is within the range of from 0.1 to 25 wt.-%, more preferably 0.5 to 22.5 wt.-%, still more preferably 1.0 to 20 wt.-%, yet more preferably 2.5 to 17.5 wt.-%, most preferably 5.0 to 15 wt.-% and in particular 7.5 to 12.5 wt.-%, based on the total weight of the pharmaceutical dosage form.

In a preferred embodiment, the plasticizer is a polyalkylene glycol having a content within the range of 5±4 wt.-%, more preferably 5±3.5 wt.-%, still more preferably 5±3 wt.-%, yet more preferably 5±2.5 wt.-%, most preferably 5±2 wt.-%, and in particular 5±1.5 wt.-%, based on the total weight of the pharmaceutical dosage form.

In another preferred embodiment, the plasticizer is a polyalkylene glycol having a content within the range of 10±8 wt.-%, more preferably 10±6 wt.-%, still more preferably 10±5 wt.-%, yet more preferably 10±4 wt.-%, most preferably 10±3 wt.-%, and in particular 10±2 wt.-%, based on the total weight of the pharmaceutical dosage form.

In still another preferred embodiment, the plasticizer is a polyalkylene glycol having a content within the range of 15±8 wt.-%, more preferably 15±6 wt.-%, still more preferably 15±5 wt.-%, yet more preferably 15±4 wt.-%, most preferably 15±3 wt.-%, and in particular 15±2 wt.-%, based on the total weight of the pharmaceutical dosage form.

Preferably, the pharmaceutical dosage form according to the invention contains an antioxidant.

Suitable antioxidants include ascorbic acid, α-tocopherol (vitamin E), butylhydroxyanisol, butylhydroxytoluene, salts of ascorbic acid (vitamin C), ascorbylic palmitate, monothio-glycerine, coniferyl benzoate, nordihydroguajaretic acid, gallus acid esters, phosphoric acid, and the derivatives thereof, such as vitamin E-succinate or vitamin E-palmitate and/or sodium bisulphite, more preferably butylhydroxytoluene (BHT) or butylhydroxyanisol (BHA) and/or α-tocopherol.

Preferably, the content of the antioxidant is within the range of from 0.001 to 5.0 wt.-%, more preferably 0.002 to 2.5 wt.-%, more preferably 0.003 to 1.5 wt.-%, still more preferably 0.005 to 1.0 wt.-%, yet more preferably 0.01 to 0.5 wt.-%, most preferably 0.05 to 0.4 wt.-% and in particular 0.1 to 0.3 wt.-%, based on the total weight of the pharmaceutical dosage form.

A particularly preferred antioxidant is α-tocopherol.

In a preferred embodiment, the content of α-tocopherol is within the range of 0.2±0.18 wt.-%, more preferably 0.2±0.15 wt.-%, still more preferably 0.2±0.12 wt.-%, yet more preferably 0.2±0.09 wt.-%, most preferably 0.2±0.06 wt.-%, and in particular 0.2±0.03 wt.-%, based on the total weight of the pharmaceutical dosage form.

In a preferred embodiment, when the pharmaceutical dosage form additionally comprises an acid, the relative weight ratio of the acid, preferably citric acid, and the antioxidant, preferably α-tocopherol, is within the range of from 10:1 to 1:10, more preferably 8:1 to 1:8, still more preferably 6:1 to 1:6, yet more preferably 5:1 to 1:4, most preferably 4:1 to 1:3 and in particular 3:1 to 1:2.

The pharmaceutical dosage form according to the invention preferably contains a free physiologically acceptable acid in an amount of from 0.001 to 5.0 wt.-%, based on the total weight of the pharmaceutical dosage form. The acid may be organic or inorganic, liquid or solid. Solid acids are preferred, particularly crystalline organic or inorganic acids.

Preferably, the acid is free. This means that the acidic functional groups of the acid are not all together constituents of a salt of the pharmacologically active ingredient, being an opioid agonist and the opioid antagonist, respectively. If the pharmacologically active ingredient, being an opioid agonist and/or the opioid antagonist is present as a salt of an acid, e.g. as hydrochloride, the pharmaceutical dosage form according to the invention preferably contains as acid another, chemically different acid which is not present as a constituent of the salt of the pharmacologically active ingredient, being an opioid agonist and the opioid antagonist, respectively. In other words, monoacids that form a salt with pharmacologically active ingredient, being an opioid agonist or opioid antagonist are not to be considered as free acids in the meaning of the invention. When acid has more than a single acidic functional group (e.g. phosphoric acid), the acid may be present as a constituent of a salt of the pharmacologically active ingredient, being an opioid agonist or the opioid antagonist, provided that at least one of the acidic functional groups of the acid is not involved in the formation of the salt, i.e. is free. Preferably, however, each and every acidic functional group of acid is not involved in the formation of a salt with pharmacologically active ingredient, being an opioid agonist and opioid antagonist. It is also possible, however, that free acid and the acid forming a salt with pharmacologically active ingredient, being an opioid agonist or opioid antagonist are identical. Under these circumstances the acid is preferably present in molar excess compared to pharmacologically active ingredient, being an opioid agonist and opioid antagonist, respectively.

In a preferred embodiment, the acid contains at least one acidic functional group (e.g. - CO₂H, -SO₃H, -PO₃H2, -OH and the like) having a pK_{A} value within the range of 2.00±1.50, more preferably 2.00±1.25, still more preferably 2.00±1.00, yet more preferably 2.00±0.75, most preferably 2.00±0.50 and in particular 2.00±0.25. In another preferred embodiment, the acid contains at least one acidic functional group having a pK_{A} value within the range of 2.25±1.50, more preferably 2.25±1.25, still more preferably 2.25±1.00, yet more preferably 2.25±0.75, most preferably 2.25±0.50 and in particular 2.25±0.25. In another preferred embodiment, the acid contains at least one acidic functional group having a pK_{A} value within the range of 2.50±1.50, more preferably 2.50±1.25, still more preferably 2.50±1.00, yet more preferably 2.50±0.75, most preferably 2.50±0.50 and in particular 2.50±0.25. In another preferred embodiment, the acid contains at least one acidic functional group having a pK_{A} value within the range of 2.75±1.50, more preferably 2.75±1.25, still more preferably 2.75±1.00, yet more preferably 2.75±0.75, most preferably 2.75±0.50 and in particular 2.75±0.25. In another preferred embodiment, the acid contains at least one acidic functional group having a pK_{A} value within the range of 3.00±1.50, more preferably 3.00±1.25, still more preferably 3.00±1.00, yet more preferably 3.00±0.75, most preferably 3.00±0.50 and in particular 3.00±0.25. In still another preferred embodiment, the acid contains at least one acidic functional group having a pK_{A} value within the range of 3.25±1.50, more preferably 3.25±1.25, still more preferably 3.25±1.00, yet more preferably 3.25±0.75, most preferably 3.25±0.50 and in particular 3.25±0.25.

In yet another preferred embodiment, the acid contains at least one acidic functional group having a pK_{A} value within the range of 4.50±1.50, more preferably 4.50±1.25, still more preferably 4.50±1.00, yet more preferably 4.50±0.75, most preferably 4.50±0.50 and in particular 4.50±0.25. In yet another preferred embodiment, the acid contains at least one acidic functional group having a pK_{A} value within the range of 4.75±1.50, more preferably 4.75±1.25, still more preferably 4.75±1.00, yet more preferably 4.75±0.75, most preferably 4.75±0.50 and in particular 4.75±0.25. In yet another preferred embodiment, the acid contains at least one acidic functional group having a pK_{A} value within the range of 5.00±1.50, more preferably 5.00±1.25, still more preferably 5.00±1.00, yet more preferably 5.00±0.75, most preferably 5.00±0.50 and in particular 5.00±0.25.

Preferably, the acid is an organic carboxylic or sulfonic acid, particularly a carboxylic acid. Multicarboxylic acids and/or hydroxy-carboxylic acids are especially preferred.

In case of multicarboxylic acids, the partial salts thereof are also to be regarded as multicarboxylic acids, e.g. the partial sodium, potassium or ammonium salts. For example, citric acid is a multicarboxylic acid having three carboxyl groups. As long as there remains at least one carboxyl group protonated (e.g. sodium dihydrogen citrate or disodium hydrogen citrate), the salt is to be regarded as a multicarboxylic acid. Preferably, however, all carboxyl groups of the multicarboxylic acid are protonated.

Preferably, the acid is of low molecular weight, i.e., not polymerized. Typically, the molecular weight of the acid is below 500 g/mol.

Examples of acids include saturated and unsaturated monocarboxylic acids, saturated and unsaturated bicarboxylic acids, tricarboxylic acids, α-hydroxyacids and β-hydroxylacids of monocarboxylic acids, α-hydroxyacids and β-hydroxyacids of bicarboxylic acids, α-hydroxyacids and β-hydroxyacids of tricarboxylic acids, ketoacids, α-ketoacids, β-ketoacids, of the polycarboxylic acids, of the polyhydroxy monocarboxylic acids, of the polyhydroxy bicarboxylic acids, of the polyhydroxy tricarboxylic acids.

Preferably, the acid is selected from the group consisting of benzenesulfonic acid, citric acid, α-glucoheptonic acid, D-gluconic acid, glycolic acid, lactic acid, malic acid, malonic acid, mandelic acid, propanoic acid, succinic acid, tartaric acid (d, l, or dl), tosic acid (toluenesulfonic acid), valeric acid, palmitic acid, pamoic acid, sebacic acid, stearic acid, lauric acid, acetic acid, adipic acid, glutaric acid, 4-chlorobenzenesulfonic acid, ethanedisulfonic acid, ethylsuccinic acid, fumaric acid, galactaric acid (mucic acid), D-glucuronic acid, 2-oxo-glutaric acid, glycerophosphoric acid, hippuric acid, isethionic acid (ethanolsulfonic acid), lactobionic acid, maleic acid, maleinic acid, 1,5-naphthalene-disulfonic acid, 2-naphthalene-sulfonic acid, pivalic acid, terephthalic acid, thiocyanic acid, cholic acid, n-dodecyl sulfate, 3-hydroxy-2-naphthoic acid, 1-hydroxy-2-naphthoic acid, oleic acid, undecylenic acid, ascorbic acid, (+)-camphoric acid, d-camphorsulfonic acid, dichloroacetic acid, ethanesulfonic acid, formic acid, methanesulfonic acid, nicotinic acid, orotic acid, oxalic acid, picric acid, L-pyroglutamic acid, saccharine, salicylic acid, gentisic acid, and/or 4-acetamidobenzoic acid.

The content of the acid is preferably within the range of from 0.001 to 5.0 wt.-%, preferably 0.005 to 2.5 wt.-%, more preferably 0.01 to 2.0 wt.-%, still more preferably 0.05 to 1.5 wt.-%, most preferably 0.1 to 1.0 wt.-% and in particular 0.2 to 0.9 wt.-%, based on the total weight of the pharmaceutical dosage form.

Preferably, the acid is a multicarboxylic acid. More preferably, the multicarboxylic acid is selected from the group consisting of citric acid, maleic acid and fumaric acid.

Citric acid is particularly preferred.

The multicarboxylic acid, preferably citric acid, may be present in its anhydrous form or as a solvate and hydrate, respectively, e.g., as monohydrate.

In a preferred embodiment, the content of the acid, preferably citric acid, is within the range of 0.2±0.18 wt.-%, more preferably 0.2±0.15 wt.-%, still more preferably 0.2±0.12 wt.-%, yet more preferably 0.2±0.09 wt.-%, most preferably 0.2±0.06 wt.-%, and in particular 0.2±0.03 wt.-%, based on the total weight of the pharmaceutical dosage form.

In another preferred embodiment, the content of the acid, preferably citric acid, is within the range of 0.3±0.18 wt.-%, more preferably 0.3±0.15 wt.-%, still more preferably 0.3±0.12 wt.-%, yet more preferably 0.3±0.09 wt.-%, most preferably 0.3±0.06 wt.-%, and in particular 0.3±0.03 wt.-%, based on the total weight of the pharmaceutical dosage form.

In still another preferred embodiment, the content of the acid, preferably citric acid, is within the range of 0.4±0.18 wt.-%, more preferably 0.4±0.15 wt.-%, still more preferably 0.4±0.12 wt.-%, yet more preferably 0.4±0.09 wt.-%, most preferably 0.4±0.06 wt.-%, and in particular 0.4±0.03 wt.-%, based on the total weight of the pharmaceutical dosage form.

In yet another preferred embodiment, the content of the acid, preferably citric acid, is within the range of 0.5±0.18 wt.-%, more preferably 0.5±0.15 wt.-%, still more preferably 0.5±0.12 wt.-%, yet more preferably 0.5±0.09 wt.-%, most preferably 0.5±0.06 wt.-%, and in particular 0.5±0.03 wt.-%, based on the total weight of the pharmaceutical dosage form.

In yet another preferred embodiment, the content of the acid, preferably citric acid, is within the range of 0.6±0.18 wt.-%, more preferably 0.6±0.15 wt.-%, still more preferably 0.6±0.12 wt.-%, yet more preferably 0.6±0.09 wt.-%, most preferably 0.6±0.06 wt.-%, and in particular 0.6±0.03 wt.-%, based on the total weight of the pharmaceutical dosage form.

In yet another preferred embodiment, the content of the acid, preferably citric acid, is within the range of 0.7±0.18 wt.-%, more preferably 0.7±0.15 wt.-%, still more preferably 0.7±0.12 wt.-%, yet more preferably 0.7±0.09 wt.-%, most preferably 0.7±0.06 wt.-%, and in particular 0.7±0.03 wt.-%, based on the total weight of the pharmaceutical dosage form.

In yet another preferred embodiment, the content of acid, preferably citric acid, is within the range of 0.8±0.18 wt.-%, more preferably 0.8±0.15 wt.-%, still more preferably 0.8±0.12 wt.-%, yet more preferably 0.8±0.09 wt.-%, most preferably 0.8±0.06 wt.-%, and in particular 0.8±0.03 wt.-%, based on the total weight of the pharmaceutical dosage form.

In yet another preferred embodiment, the content of the acid, preferably citric acid, is within the range of 0.85±0.18 wt.-%, more preferably 0.85±0.15 wt.-%, still more preferably 0.85±0.12 wt.-%, yet more preferably 0.85±0.09 wt.-%, most preferably 0.85±0.06 wt.-%, and in particular 0.85±0.03 wt.-%, based on the total weight of the pharmaceutical dosage form.

In still another preferred embodiment, the content of the acid, preferably citric acid, is within the range of 0.9±0.18 wt.-%, more preferably 0.9±0.15 wt.-%, still more preferably 0.9±0.12 wt.-%, yet more preferably 0.9±0.09 wt.-%, most preferably 0.9±0.06 wt.-%, and in particular 0.9±0.03 wt.-%, based on the total weight of the pharmaceutical dosage form.

In a further preferred embodiment, the content of the acid, preferably citric acid, is within the range of 1.0±0.18 wt.-%, more preferably 1.0±0.15 wt.-%, still more preferably 1.0±0.12 wt.-%, yet more preferably 1.0±0.09 wt.-%, most preferably 1.0±0.06 wt.-%, and in particular 1.0±0.03 wt.-%, based on the total weight of the pharmaceutical dosage form.

The pharmaceutical dosage form according to the invention may also contain a natural, semi-synthetic or synthetic wax. Waxes with a softening point of at least 50 °C, more preferably 60 °C are preferred. Carnauba wax and beeswax are particularly preferred, especially carnauba wax.

Preferably, the pharmaceutical dosage form according to the invention contains a coating, preferably a film-coating. Suitable coating materials are known to the skilled person. Suitable coating materials are commercially available, e.g. under the trademarks Opadry® and Eudragit®.

Examples of suitable materials include cellulose esters and cellulose ethers, such as methylcellulose (MC), hydroxypropylmethylcellulose (HPMC), hydroxypropylcellulose (HPC), hydroxyethylcellulose (HEC), sodium carboxymethylcellulose (Na-CMC), ethylcellulose (EC), cellulose acetate phthalate (CAP), hydroxypropylmethylcellulose phthalate (HPMCP); poly(meth)acrylates, such as aminoalkylmethacrylate copolymers, ethylacrylate methylmethacrylate copolymers, methacrylic acid methylmethacrylate copolymers, methacrylic acid methylmethacrylate copolymers; vinyl polymers, such as polyvinylpyrrolidone, polyvinyl-acetatephthalate, polyvinyl alcohol, polyvinylacetate; and natural film formers, such as shellack.

In a particularly preferred embodiment, the coating is water-soluble. In a preferred embodiment, the coating is based on polyvinyl alcohol, such as polyvinyl alcohol-part. hydrolyzed, and may additionally contain polyethylene glycol, such as macrogol 3350, and/or pigments. In another preferred embodiment, the coating is based on hydroxypropylmethylcellulose, preferably hypromellose type 2910 having a viscosity of 3 to 15 mPas.

The coating of the pharmaceutical dosage form can increase its storage stability.

The coating can be resistant to gastric juices and dissolve as a function of the pH value of the release environment. By means of this coating, it is possible to ensure that the pharmaceutical dosage form according to the invention passes through the stomach undissolved and the active compound is only released in the intestines. The coating which is resistant to gastric juices preferably dissolves at a pH value of between 5 and 7.5. Corresponding materials and methods for the delayed release of active compounds and for the application of coatings which are resistant to gastric juices are known to the person skilled in the art, for example from "Coated Pharmaceutical dosage forms - Fundamentals, Manufacturing Techniques, Biopharmaceutical Aspects, Test Methods and Raw Materials" by Kurt H. Bauer, K. Lehmann, Hermann P. Osterwald, Rothgang, Gerhart, 1st edition, 1998, Medpharm Scientific Publishers.

The pharmaceutical dosage form according to the invention is preferably tamper-resistant. Preferably, tamper-resistance is achieved based on the mechanical properties of the pharmaceutical dosage form so that comminution is avoided or at least substantially impeded. According to the invention, the term comminution means the pulverization of the pharmaceutical dosage form using conventional means usually available to an abuser, for example a pestle and mortar, a hammer, a mallet or other conventional means for pulverizing under the action of force. Thus, tamper-resistance preferably means that pulverization of the pharmaceutical dosage form using conventional means is avoided or at least substantially impeded.

Preferably, the mechanical properties of the pharmaceutical dosage form according to the invention, particularly its breaking strength, substantially rely on the presence and spatial distribution of the polyalkylene oxide, although its mere presence does typically not suffice in order to achieve said properties. The advantageous mechanical properties of the pharmaceutical dosage form according to the invention may not automatically be achieved by simply processing pharmacologically active ingredient, being an opioid agonist, opioid antagonist, polyalkylene oxide, and optionally further excipients by means of conventional methods for the preparation of pharmaceutical dosage forms. In fact, usually suitable apparatuses must be selected for the preparation and critical processing parameters must be adjusted, particularly pressure/force, temperature and time. Thus, even if conventional apparatuses are used, the process protocols usually must be adapted in order to meet the required criteria.

Furthermore, tamper-resistance is achieved based on the poor solubility properties of the pharmaceutical dosage form in alcohol, especially ethanol, thereby effectively preventing alcohol dose dumping.

The pharmaceutical dosage form according to the invention has a breaking strength of at least 300 N, preferably at least 400 N, more preferably at least 500 N, still more preferably at least 750 N, yet more preferably at least 1000 N, most preferably at least 1250 N and in particular at least 1500 N.

The "breaking strength" (resistance to crushing) of a pharmaceutical dosage form is known to the skilled person. In this regard it can be referred to, e.g., W.A. Ritschel, Die Tablette, 2. Auflage, Editio Cantor Verlag Aulendorf, 2002; H Liebermann et al., Pharmaceutical dosage forms: Tablets, Vol. 2, Informa Healthcare; 2 edition, 1990; and Encyclopedia of Pharmaceutical Technology, Informa Healthcare; 1 edition.

For the purpose of the specification, the breaking strength is preferably defined as the amount of force that is necessary in order to fracture the pharmaceutical dosage form (= breaking force). Therefore, for the purpose of the specification the pharmaceutical dosage form does preferably not exhibit the desired breaking strength when it breaks, i.e., is fractured into at least two independent parts that are separated from one another. In another preferred embodiment, however, the pharmaceutical dosage form is regarded as being broken if the force decreases by 25% (threshold value) of the highest force measured during the measurement (see below).

The pharmaceutical dosage forms according to the invention are distinguished from conventional pharmaceutical dosage forms in that, due to their breaking strength, they cannot be pulverized by the application of force with conventional means, such as for example a pestle and mortar, a hammer, a mallet or other usual means for pulverization, in particular devices developed for this purpose (tablet crushers). In this regard "pulverization" preferably means crumbling into small particles that would immediately release the pharmacologically active compound (A) in a suitable medium. Avoidance of pulverization virtually rules out oral or parenteral, in particular intravenous or nasal abuse.

Conventional tablets typically have a breaking strength well below 200 N in any direction of extension. The breaking strength of conventional round tablets may be estimated according to the following empirical formula: Breaking Strength [in N] = 10 x Diameter Of The Tablet [in mm]. Thus, according to said empirical formula, a round tablet having a breaking strength of at least 300 N would require a diameter of at least 30 mm). Such a tablet, however, could not be swallowed. The above empirical formula preferably does not apply to the pharmaceutical dosage forms of the invention, which are not conventional but rather special.

Further, the actual mean chewing force is about 220 N (cf., e.g., P.A. Proeschel et al., J Dent Res, 2002, 81(7), 464-468). This means that conventional tablets having a breaking strength well below 200 N may be crushed upon spontaneous chewing, whereas the pharmaceutical dosage forms according to the invention may not.

Still further, when applying a gravitational acceleration of about 9.81 m/s2, 300 N correspond to a gravitational force of more than 30 kg, i.e. the pharmaceutical dosage forms according to the invention can preferably withstand a weight of more than 30 kg without being pulverized.

Methods for measuring the breaking strength of a pharmaceutical dosage form are known to the skilled artisan. Suitable devices are commercially available.

For example, the breaking strength (resistance to crushing) can be measured in accordance with the Eur. Ph. 5.0, 2.9.8 or 6.0, 2.09.08 "Resistance to Crushing of Tablets". The test is intended to determine, under defined conditions, the resistance to crushing of tablets, measured by the force needed to disrupt them by crushing. The apparatus consists of 2 jaws facing each other, one of which moves towards the other. The flat surfaces of the jaws are perpendicular to the direction of movement. The crushing surfaces of the jaws are flat and larger than the zone of contact with the tablet. The apparatus is calibrated using a system with a precision of 1 Newton. The tablet is placed between the jaws, taking into account, where applicable, the shape, the break-mark and the inscription; for each measurement the tablet is oriented in the same way with respect to the direction of application of the force (and the direction of extension in which the breaking strength is to be measured). The measurement is carried out on 10 tablets, taking care that all fragments of tablets have been removed before each determination. The result is expressed as the mean, minimum and maximum values of the forces measured, all expressed in Newton.

A similar description of the breaking strength (breaking force) can be found in the USP. The breaking strength can alternatively be measured in accordance with the method described therein where it is stated that the breaking strength is the force required to cause a tablet to fail (i.e., break) in a specific plane. The tablets are generally placed between two plates, one of which moves to apply sufficient force to the tablet to cause fracture. For conventional, round (circular cross-section) tablets, loading occurs across their diameter (sometimes referred to as diametral loading), and fracture occurs in the plane. The breaking force of tablets is commonly called hardness in the pharmaceutical literature; however, the use of this term is misleading. In material science, the term hardness refers to the resistance of a surface to penetration or indentation by a small probe. The term crushing strength is also frequently used to describe the resistance of tablets to the application of a compressive load. Although this term describes the true nature of the test more accurately than does hardness, it implies that tablets are actually crushed during the test, which is often not the case.

Alternatively, the breaking strength (resistance to crushing) can be measured in accordance with WO 2005/ 016313, WO 2005/016314, and WO 2006/082099, which can be regarded as a modification of the method described in the Eur. Ph. The apparatus used for the measurement is preferably a "Zwick Z 2.5" materials tester, Fₘₐₓ = 2.5 kN with a maximum draw of 1150 mm, which should be set up with one column and one spindle, a clearance behind of 100 mm and a test speed adjustable between 0.1 and 800 mm/min together with testControl software. Measurement is performed using a pressure piston with screw-in inserts and a cylinder (diameter 10 mm), a force transducer, Fₘₐₓ. 1 kN, diameter = 8 mm, class 0.5 from 10 N, class 1 from 2 N to ISO 7500-1, with manufacturer's test certificate M according to DIN 55350-18 (Zwick gross force Fₘₐₓ = 1.45 kN) (all apparatus from Zwick GmbH & Co. KG, Ulm, Germany) with Order No BTC-FR 2.5 TH. D09 for the tester, Order No BTC-LC 0050N. P01 for the force transducer, Order No BO 70000 S06 for the centring device.

In a preferred embodiment of the invention, the breaking strength is measured by means of a breaking strength tester e.g. Sotax®, type HT100 or type HT1 (Allschwil, Switzerland). Both, the Sotax® HT100 and the Sotax® HT1 can measure the breaking strength according to two different measurement principles: constant speed (where the test jaw is moved at a constant speed adjustable from 5-200 mm/min) or constant force (where the test jaw increases force linearly adjustable from 5-100 N/sec). In principle, both measurement principles are suitable for measuring the breaking strength of the pharmaceutical dosage form according to the invention. Preferably, the breaking strength is measured at constant speed, preferably at a constant speed of 120 mm/min.

In a preferred embodiment, the pharmaceutical dosage form is regarded as being broken if it is fractured into at least two separate pieces.

The pharmaceutical dosage form according to the invention preferably exhibits mechanical strength over a wide temperature range, in addition to the breaking strength (resistance to crushing) optionally also sufficient hardness, impact resistance, impact elasticity, tensile strength and/or modulus of elasticity, optionally also at low temperatures (e.g. below -24 °C, below -40 °C or in liquid nitrogen), for it to be virtually impossible to pulverize by spontaneous chewing, grinding in a mortar, pounding, etc. Thus, preferably, the comparatively high breaking strength of the pharmaceutical dosage form according to the invention is maintained even at low or very low temperatures, e.g., when the pharmaceutical dosage form is initially chilled to increase its brittleness, for example to temperatures below -25°C, below -40 °C or even in liquid nitrogen.

The pharmaceutical dosage form according to the invention is characterized by a certain degree of breaking strength. This does not mean that the pharmaceutical dosage form must also exhibit a certain degree of hardness. Hardness and breaking strength are different physical properties. Therefore, the tamper resistance of the pharmaceutical dosage form does not necessarily depend on the hardness of the pharmaceutical dosage form. For instance, due to its breaking strength, impact strength, elasticity modulus and tensile strength, respectively, the pharmaceutical dosage form can preferably be deformed, e.g. plastically, when exerting an external force, for example using a hammer, but cannot be pulverized, i.e., crumbled into a high number of fragments. In other words, the pharmaceutical dosage form according to the invention is characterized by a certain degree of breaking strength, but not necessarily also by a certain degree of form stability.

Therefore, in the meaning of the specification, a pharmaceutical dosage form that is deformed when being exposed to a force in a particular direction of extension but that does not break (plastic deformation or plastic flow) is preferably to be regarded as having the desired breaking strength in said direction of extension.

In a preferred embodiment, when
(i) stirring an intact pharmaceutical dosage form according to the invention for 30 minutes in 30 mL of purified water at ambient temperature;
(ii) separating the overhead liquid solution from the remainder, e.g., by means of a syringe, equipped with a cigarette filter and a canula, 0.80 x 40 mm BL/LB; 21 G x 1 1/2", and
(iii) determining the pharmacologically active compound content in the drawn liquid by HPLC analysis;
the content of extracted pharmacologically active compound (in each case pharmacologically active ingredient, being an opioid agonist and opioid antagonist) in the overhead liquid amounts to at most 80 wt.-%, 77.5 wt.-%, 75 wt.-%, or 72.5 wt.-%, more preferably at most 70 wt.-%, 67.5 wt.-%, 65 wt.-%, or 62.5 wt.-%, still more preferably at most 60 wt.-%, 57.5 wt.-%, 55 wt.-%, or 52.5 wt.-%, yet more preferably at most 50 wt.-%, 49 wt.-%, 48 wt.-%, or 47 wt.-%, even more preferably at most 46 wt.-%, 45 wt.-%, 44 wt.-%, or 43 wt.-%, most more preferably at most 42 wt.-%, 41 wt.-%, 40 wt.-%, or 39 wt.-%, and in particular at most 38 wt.-%, 37 wt.-%, 36 wt.-%, or 35 wt.-%, relative to the original total content of the pharmacologically active compound in the pharmaceutical dosage form, i.e. before it was subjected to the extraction test. Preferably, the quantity of extracted pharmacologically active ingredient, being an opioid agonist and the quantity of extracted opioid antagonist do not deviate from one another by more than 3.0 wt.-%, more preferably not more than 2.8 wt.-%, still more preferably not more than 2.6 wt.-%, yet more preferably not more than 2.4 wt.-%, even more preferably not more than 2.2 wt.-%, most preferably not more than 2.0 wt.-% and in particular not more than 1.8 wt.-%, wherein the percentages mean absolute values with respect to the amount of pharmacologically active ingredient, being an opioid agonist and antagonist, respectively, that was originally contained in the pharmaceutical dosage form.

In another preferred embodiment, when
(i) putting an intact pharmaceutical dosage form according to the invention into 30 mL of boiling purified water (∼100 °C) and allowing the water to cool down over 30 minutes;
(ii) supplementing lost water, if any;
(iii) separating a defined quantity of the overhead liquid solution from the remainder, e.g., by means of a syringe, equipped with a canula, 0.80 x 40 mm BL/LB; 21 G x 1 1/2", and
(iv) determining the pharmacologically active compound content in the drawn liquid by HPLC analysis;
the content of extracted pharmacologically active compound (in each case pharmacologically active ingredient, being an opioid agonist and opioid antagonist) in the overhead liquid amounts to at most 21.5 wt.-%, 21.0 wt.-%, 20.5 wt.-%, or 20.0 wt.-%, more preferably at most 19.5 wt.-%, 19.0 wt.-%, 18.5 wt.-%, or 18.0 wt.-%, still more preferably at most 17.5 wt.-%, 17.0 wt.-%, 16.5 wt.-%, or 16.0 wt.-%, yet more preferably at most 15.5 wt.-%, 15.0 wt.-%, 14.5 wt.-%, or 14.0 wt.-%, even more preferably at most 13.5 wt.-%, 13.0 wt.-%, 12.5 wt.-%, or 12.0 wt.-%, most more preferably at most 11.5 wt.-%, 11.0 wt.-%, 10.5 wt.-%, or 10.0 wt.-%, and in particular at most 9.5 wt.-%, 9.0 wt.-%, 8.5 wt.-%, or 8.0 wt.-%, relative to the original total content of the pharmacologically active compound in the pharmaceutical dosage form, i.e. before it was subjected to the extraction test. Preferably, the quantity of extracted pharmacologically active ingredient, being an opioid agonist and the quantity of extracted opioid antagonist do not deviate from one another by more than 3.0 wt.-%, more preferably not more than 2.8 wt.-%, still more preferably not more than 2.6 wt.-%, yet more preferably not more than 2.4 wt.-%, even more preferably not more than 2.2 wt.-%, most preferably not more than 2.0 wt.-% and in particular not more than 1.8 wt.-%, wherein the percentages mean absolute values with respect to the amount of pharmacologically active ingredient, being an opioid agonist and antagonist, respectively, that was originally contained in the pharmaceutical dosage form.

In still another preferred embodiment, when
(i) stirring an intact pharmaceutical dosage form according to the invention for 30 minutes in 30 mL of aqueous ethanol (40% (v/v)) (ambient temperature);
(ii) separating the overhead liquid solution from the remainder, e.g., by means of a syringe, equipped with a cigarette filter and a canula, 0.80 x 40 mm BL/LB; 21 G x 1 1/2", and
(iii) determining the pharmacologically active compound content in the drawn liquid by HPLC analysis;
the content of extracted pharmacologically active compound (in each case pharmacologically active ingredient, being an opioid agonist and opioid antagonist) in the overhead liquid amounts to at most 18.5 wt.-%, 18.0 wt.-%, 17.5 wt.-%, or 17.0 wt.-%, more preferably at most 16.5 wt.-%, 16.0 wt.-%, 15.5 wt.-%, or 15.0 wt.-%, still more preferably at most 14.5 wt.-%, 14.0 wt.-%, 13.5 wt.-%, or 13.0 wt.-%, yet more preferably at most 12.5 wt.-%, 12.0 wt.-%, 11.5 wt.-%, or 11.0 wt.-%, even more preferably at most 10.5 wt.-%, 10.0 wt.-%, 9.5 wt.-%, or 9.0 wt.-%, most more preferably at most 8.5 wt.-%, 8.0 wt.-%, 7.5 wt.-%, or 7.0 wt.-%, and in particular at most 6.5 wt.-%, 6.0 wt.-%, 5.5 wt.-%, or 5.0 wt.-%, relative to the original total content of the pharmacologically active compound in the pharmaceutical dosage form, i.e. before it was subjected to the extraction test. Preferably, the quantity of extracted pharmacologically active ingredient, being an opioid agonist and the quantity of extracted opioid antagonist do not deviate from one another by more than 3.0 wt.-%, more preferably not more than 2.8 wt.-%, still more preferably not more than 2.6 wt.-%, yet more preferably not more than 2.4 wt.-%, even more preferably not more than 2.2 wt.-%, most preferably not more than 2.0 wt.-% and in particular not more than 1.8 wt.-%, wherein the percentages mean absolute values with respect to the amount of pharmacologically active ingredient, being an opioid agonist and antagonist, respectively, that was originally contained in the pharmaceutical dosage form.

Preferably, when a pharmaceutical dosage form according to the invention is treated with a commercial coffee mill, preferably type Bosch MKM6000, 180W, Typ KM13 for 2 minutes, 42±17.5 wt.-%, more preferably 42±15 wt.-%, still more preferably 42±12.5 wt.-%, yet more preferably 42±10 wt.-%, even more preferably 42±7.5 wt.-%, most preferably 42±5 wt.-%, and in particular 42±2.5 wt.-%, of the total weight of the thus obtained material passes a sieve having a mesh size of 125 µm but does not pass a sieve having a mesh size of 1.000 mm.

Preferably, when a pharmaceutical dosage form according to the invention is treated with a commercial coffee mill, preferably type Bosch MKM6000, 180W, Typ KM13, for 2 minutes, 57±17.5 wt.-%, more preferably 57±15 wt.-%, still more preferably 57±12.5 wt.-%, yet more preferably 57±10 wt.-%, even more preferably 57±7.5 wt.-%, most preferably 57±5 wt.-%, and in particular 57±2.5 wt.-%, of the total weight of the thus obtained material does not pass a sieve having a mesh size of 1.000 mm.

Preferably, when a pharmaceutical dosage form according to the invention is treated with a commercial coffee mill, preferably type Bosch MKM6000, 180W, Typ KM13, for 2 minutes, at least 50 wt.-%, more preferably at least 55 wt.-%, still more preferably at least 60 wt.-%, yet more preferably at least 65 wt.-%, even more preferably at least 70 wt.-%, most preferably at least 75 wt.-%, and in particular at least 80 wt.-%, of the total weight of the thus obtained material does not pass a sieve having a mesh size of 1.000 mm.

Preferably, the pharmaceutical dosage form for oral administration
- has a breaking strength of at least 400 N, more preferably at least 500 N, still more preferably at least 750 N, yet more preferably at least 1000 N, most preferably at least 1500 N; and/or
- comprises an opioid agonist selected from oxycodone and the physiologically acceptable salts thereof; and/or
- comprises an opioid antagonist selected from naloxone and the physiologically acceptable salts thereof, and an aversive agent; and/or
- is configured for oral administration twice daily; and/or
- contains at least 30 wt.-%, more preferably at least 35 wt.-%, still more preferably at least 40 wt.-% of a polyalkylene oxide having an average molecular weight of at least 500,000 g/mol, more preferably at least 1,000,000 g/mol, relative to the total weight of the pharmaceutical dosage form; and/or
- contains as anionic polymer an optionally cross-linked homo- or copolymer of acrylic acid; and/or
- contains a plasticizer, preferably polyethylene glycol; and/or
- contains an antioxidant, preferably α-tocopherol; and/or
- optionally, contains a free acid, preferably citric acid; and/or
- optionally, contains an additional matrix polymer, preferably a cellulose ether, more preferably HPMC.

The pharmaceutical dosage form according to the invention may be produced by different processes, the particularly preferred of which are explained in greater detail below. Several suitable processes have already been described in the prior art. In this regard it can be referred to, e.g., WO 2005/ 016313, WO 2005/016314, WO 2005/063214, WO 2005/102286, WO 2006/002883, WO 2006/002884, WO 2006/002886, WO 2006/082097, and WO 2006/082099.

The invention also relates to pharmaceutical dosage forms that are obtainable by any of the processes described here below.

In general, the process for the production of the pharmaceutical dosage form according to the invention preferably comprises the following steps:
a) mixing all ingredients;
b) optionally pre-forming the mixture obtained from step (a), preferably by applying heat and/or force to the mixture obtained from step (a), the quantity of heat supplied preferably not being sufficient to heat the polyalkylene oxide up to its softening point;
c) hardening the mixture by applying heat and force, it being possible to supply the heat during and/or before the application of force and the quantity of heat supplied being sufficient to heat the polyalkylene oxide at least up to its softening point;
d) optionally singulating the hardened mixture;
e) optionally shaping the pharmaceutical dosage form; and
f) optionally providing a film coating.

Heat may be supplied directly, e.g. by contact or by means of hot gas such as hot air, or with the assistance of ultrasound. Force may be applied and/or the pharmaceutical dosage form may be shaped for example by direct tabletting or with the assistance of a suitable extruder, particularly by means of a screw extruder equipped with two screws (twin-screw-extruder) or by means of a planetary gear extruder.

The final shape of the pharmaceutical dosage form may either be provided during the hardening of the mixture by applying heat and force (step (c)) or in a subsequent step (step (e)). In both cases, the mixture of all components is preferably in the plastified state, i.e. preferably, shaping is performed at a temperature at least above the softening point of the polyalkylene oxide. However, extrusion at lower temperatures, e.g. ambient temperature, is also possible and may be preferred.

Shaping can be performed, e.g., by means of a tabletting press comprising die and punches of appropriate shape.

A particularly preferred process for the manufacture of the pharmaceutical dosage form of the invention involves hot-melt extrusion. In this process, the pharmaceutical dosage form according to the invention is produced by thermoforming with the assistance of an extruder, preferably without there being any observable consequent discoloration of the extrudate. It has been surprisingly found that acid is capable of suppressing discoloration. In the absence of acid, the extrudate tends to develop beige to yellowish coloring whereas in the presence of acid the extrudates are substantially colorless, i.e. white.

This process is characterized in that
a) all components are mixed,
b) the resultant mixture is heated in the extruder at least up to the softening point of the polyalkylene oxide and extruded through the outlet orifice of the extruder by application of force,
c) the still plastic extrudate is singulated and formed into the pharmaceutical dosage form or
d) the cooled and optionally reheated singulated extrudate is formed into the pharmaceutical dosage form.

Mixing of the components according to process step a) may also proceed in the extruder.

The components may also be mixed in a mixer known to the person skilled in the art. The mixer may, for example, be a roll mixer, shaking mixer, shear mixer or compulsory mixer.

Before blending with the remaining components, polyalkylene oxide is preferably provided according to the invention with an antioxidant, preferably α-tocopherol. This may proceed by mixing the two components, the polyalkylene oxide and the antioxidant, preferably by dissolving or suspending the antioxidant in a highly volatile solvent and homogeneously mixing this solution or suspension with polyalkylene oxide and removing the solvent by drying, preferably under an inert gas atmosphere.

The, preferably molten, mixture which has been heated in the extruder at least up to the softening point of polyalkylene oxide is extruded from the extruder through a die with at least one bore.

The process according to the invention requires the use of suitable extruders, preferably screw extruders. Screw extruders which are equipped with two screws (twin-screw-extruders) are particularly preferred.

The extrusion is preferably performed so that the expansion of the strand due to extrusion is not more than 30%, i.e. that when using a die with a bore having a diameter of e.g. 6 mm, the extruded strand should have a diameter of not more than 8 mm. More preferably, the expansion of the strand is not more than 25%, still more preferably not more than 20%, most preferably not more than 15% and in particular not more than 10%.

Preferably, extrusion is performed in the absence of water, i.e., no water is added. However, traces of water (e.g., caused by atmospheric humidity) may be present.

The extruder preferably comprises at least two temperature zones, with heating of the mixture at least up to the softening point of the polyalkylene oxide proceeding in the first zone, which is downstream from a feed zone and optionally mixing zone. The throughput of the mixture is preferably from 1.0 kg to 15 kg/hour. In a preferred embodiment, the throughput is from 1 to 3.5 kg/hour. In another preferred embodiment, the throughput is from 4 to 15 kg/hour.

In a preferred embodiment, the die head pressure is within the range of from 25 to 100 bar. The die head pressure can be adjusted inter alia by die geometry, temperature profile and extrusion speed.

The die geometry or the geometry of the bores is freely selectable. The die or the bores may accordingly exhibit a round, oblong or oval cross-section, wherein the round cross-section preferably has a diameter of 0.1 mm to 15 mm and the oblong cross-section preferably has a maximum lengthwise extension of 21 mm and a crosswise extension of 10 mm. Preferably, the die or the bores have a round cross-section. The casing of the extruder used according to the invention may be heated or cooled. The corresponding temperature control, i.e. heating or cooling, is arranged in such a way that the mixture to be extruded exhibits at least an average temperature (product temperature) corresponding to the softening temperature of the polyalkylene oxide and does not rise above a temperature at which the pharmacologically active ingredient, being an opioid agonist to be processed may be damaged. Preferably, the temperature of the mixture to be extruded is adjusted to below 180 °C, preferably below 150 °C, but at least to the softening temperature of polyalkylene oxide. Typical extrusion temperatures are 120 °C and 130 °C.

In a preferred embodiment, the extruder torque is within the range of from 30 to 95%. Extruder torque can be adjusted inter alia by die geometry, temperature profile and extrusion speed.

After extrusion of the molten mixture and optional cooling of the extruded strand or extruded strands, the extrudates are preferably singulated. This singulation may preferably be performed by cutting up the extrudates by means of revolving or rotating knives, water jet cutters, wires, blades or with the assistance of laser cutters.

Preferably, intermediate or final storage of the optionally singulated extrudate or the final shape of the pharmaceutical dosage form according to the invention is performed under oxygen-free atmosphere which may be achieved, e.g., by means of oxygen-scavengers.

The singulated extrudate may be press-formed into tablets in order to impart the final shape to the pharmaceutical dosage form.

The application of force in the extruder onto the at least plasticized mixture is adjusted by controlling the rotational speed of the conveying device in the extruder and the geometry thereof and by dimensioning the outlet orifice in such a manner that the pressure necessary for extruding the plasticized mixture is built up in the extruder, preferably immediately prior to extrusion. The extrusion parameters which, for each particular composition, are necessary to give rise to a pharmaceutical dosage form with desired mechanical properties, may be established by simple preliminary testing.

For example but not limiting, extrusion may be performed by means of a twin-screw-extruder type ZSE 18 or ZSE27 (Leistritz, Nürnberg, Germany), screw diameters of 18 or 27 mm. Screws having eccentric ends may be used. A heatable die with a round bore having a diameter of 4, 5, 6, 7, 8, or 9 mm may be used. The extrusion parameters may be adjusted e.g. to the following values: rotational speed of the screws: 120 Upm; delivery rate 1 kg/h for a ZSE 18 or 8-10 kg/h for a ZSE27; product temperature: in front of die 125 °C and behind die 135 °C; and jacket temperature: 110 °C.

Preferably, extrusion is performed by means of twin-screw-extruders or planetary-gear-extruders, twin-screw extruders (co-rotating or contra-rotating) being particularly preferred.

The pharmaceutical dosage form according to the invention is preferably produced by thermoforming with the assistance of an extruder without any observable consequent discoloration of the extrudates.

The process for the preparation of the pharmaceutical dosage form according to the invention is preferably performed continuously. Preferably, the process involves the extrusion of a homogeneous mixture of all components. It is particularly advantageous if the thus obtained intermediate, e.g. the strand obtained by extrusion, exhibits uniform properties. Particularly desirable are uniform density, uniform distribution of the active compound, uniform mechanical properties, uniform porosity, uniform appearance of the surface, etc. Only under these circumstances the uniformity of the pharmacological properties, such as the stability of the release profile, may be ensured and the amount of rejects can be kept low.

A further aspect of the invention relates to the use of an pharmacologically active ingredient, being an opioid agonist in combination with an opioid antagonist for the manufacture of the pharmaceutical dosage form as described above for the treatment of pain, preferably moderate to severe pain such as moderate to severe low back pain.

A further aspect of the invention relates to the use of a pharmaceutical dosage form as described above for avoiding or hindering the abuse of the pharmacologically active ingredient, being an opioid agonist contained therein.

A further aspect of the invention relates to the use of a pharmaceutical dosage form as described above for avoiding or hindering the unintentional overdose of the pharmacologically active ingredient, being an opioid agonist contained therein.

In this regard, the invention also relates to the use of a pharmacologically active ingredient, being an opioid agonist as described above and/or a opioid antagonist as described above and/or a polyalkylene oxide as described above for the manufacture of the pharmaceutical dosage form according to the invention for the prophylaxis and/or the treatment of a disorder, thereby preventing an overdose of the pharmacologically active ingredient, being an opioid agonist, particularly due to comminution of the pharmaceutical dosage form by mechanical action.

Further, the invention relates to a method for the prophylaxis and/or the treatment of a disorder comprising the administration of the pharmaceutical dosage form according to the invention, thereby preventing an overdose of the pharmacologically active ingredient, being an opioid agonist, particularly due to comminution of the pharmaceutical dosage form by mechanical action. Preferably, the mechanical action is selected from the group consisting of chewing, grinding in a mortar, pounding, and using apparatuses for pulverizing conventional pharmaceutical dosage forms.

The following examples further illustrate the invention but are not to be construed as limiting its scope.

### General procedure:

Polyethylene oxide, α-tocopherol, oxycodone hydrochloride, naloxone hydrochloride and all other excipients were weighted and sieved to each other.

The powder was mixed and dosed gravimetrically to an extruder. Hot-melt extrusion was performed by means of a twin screw extruder of type ZSE18 PH 40D (Leistritz, Nürnberg, Germany) that was equipped with medium shear screws and a heatable round die having a diameter of 5 or 7mm. Extrusion was performed at 100 rpm at a dosing rate of 1 kg/h.

The hot extrudate was cooled by ambient air and the cooled extrusion strand was comminuted to cut pieces. The cut pieces were shaped by means of an excenter press which was equipped with punches of various size and shape.

The breaking strength of the pharmaceutical dosage forms was measured by means of a Sotax® HT100. A tablet was regarded as failing the breaking strength test when during the measurement the force dropped below the threshold value of 25% of the maximum force that was observed during the measurement, regardless of whether the dosage form was fractured into separate pieces or not. All values are given as a mean of 10 measurements.

The *in vitro* release profile of the pharmacologically active ingredient (Oxycodone HCI and Naloxone HCl) was measured in 600 ml of blank FeSSIF (pH 5.0) at temperature of 37°C with sinker (type 1 or 2). The rotation speed of the paddle was adjusted to 150/min. The pharmacologically active ingredient was detected by means of a spectrometric measurement with a wavelength of 218 nm.

### Example I:

### Composition of formulations

The following compositions were prepared and hot-melt extruded:

| | C-1 | C-2 | I-1 |
|---|---|---|---|
| Oxycodone HCl | 16.00% | 16.00% | 16.00% |
| Naloxone HCl | 8.00% | 8.00% | 8.00% |
| Polyethylene oxide M_{w} 7,000,000 | 62.60% | 45.00% | 40.00% |
| HPMC | 8.50% | 15.00% | 15.00% |
| PEG6000 | 3.75% | 15.00 | 15.00 |
| Citric acid, anhydrous | 0.22% | 0.20% | 0.20% |
| α-Tocopherol | 0.93% | 0.80% | 0.80% |
| Carbopol 71G | - | - | 5.00% |
| tablet weight | 250.00 mg | 250.00 mg | 250.00 mg |

### Tablet formats

Tablets were manufactured from the crude extrudates by means of round punch and oblong punch, respectively (no engraving). Tablets having the following formats were prepared from the cut extrudates by means of the corresponding punches:

| # | ø of extrusion die | tablets | punch format |
|---|---|---|---|
| C-1 | 7.0 mm | C-1₇^{round} | 9 mm round |
| | | | radius of curvature 7.2 mm |
| | | C-1₇^{biconvex} | 9 mm round |
| | | | R=15/1 biconvex |
| | 5.0 mm | C-1₅^{oblong} | oblong |
| | | | 6x15 mm |
| C-2 | 7.0 mm | C-2₇^{round} | 9 mm round |
| | | | radius of curvature 7.2 mm |
| | | C-2₇^{biconvex} | 9 mm round |
| | | | R=15/1 biconvex |
| | 5.0 mm | C-2₅^{oblong} | oblong |
| | | | 6x15 mm |
| I-1 | 7.0 mm | I-1₇^{round} | 9 mm round |
| | | | radius of curvature 7.2 mm |
| | | I-1₇ ^{biconvex} | 9 mm round |
| | | | R=15/1 biconvex |
| | 5.0 mm | I-1₅^{blong} | oblong |
| | | | 6x15 mm |

### Analytical tests - in vitro release

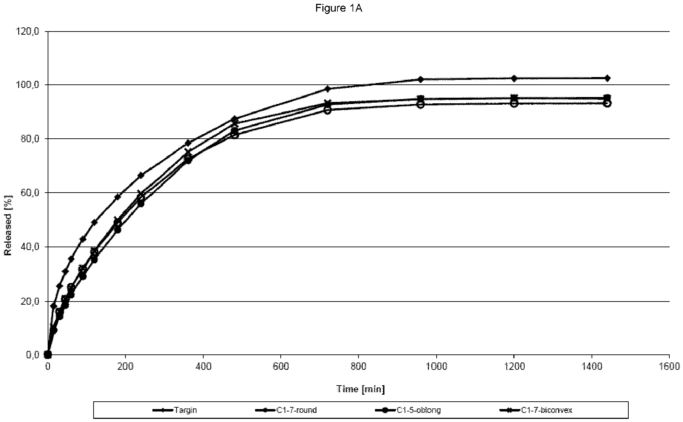

Figure 1A shows the *in vitro* release profile of oxycodone from the tablets C-1₇^{round}, C-1₇^{biconvex}, and C-1₅^{oblong} (different tablet formats) as manufactured from composition C-1 in comparison to commercial Targin® tablets.

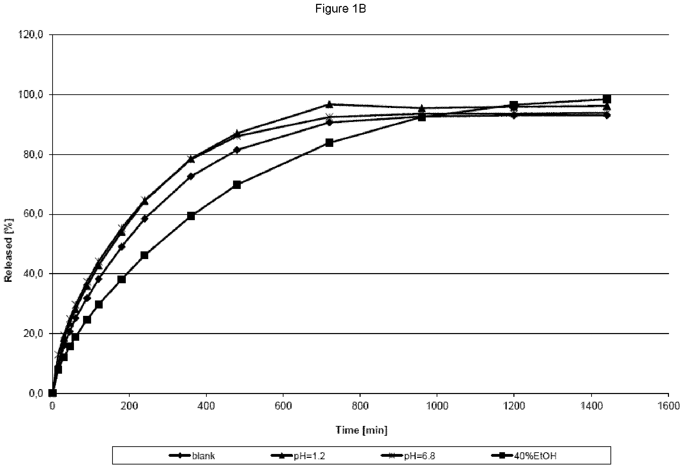

Figure 1B shows the *in vitro* release profile of oxycodone from the tablets as manufactured from composition C-1₅^{oblong} in different release media (blank Fessif, pH 1.2, pH 6.8, and aqueous ethanol 40% (v/v)).

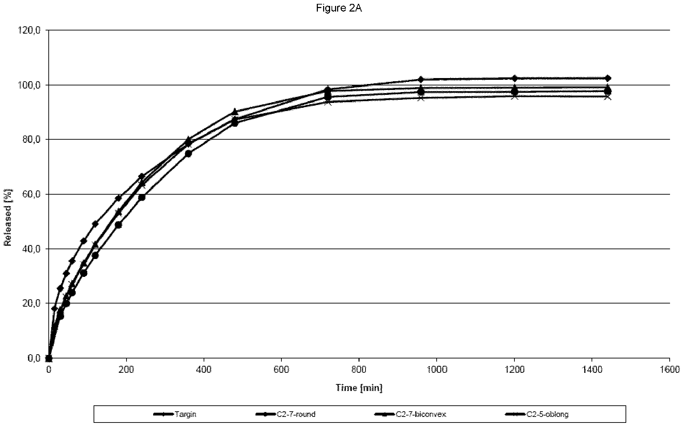

Figure 2A shows the *in vitro* release profile of oxycodone from the tablets C-2₇^{round}, C-2₇^{biconvex}, and C-2₅^{oblong} (different tablet formats) as manufactured from composition C-2 in comparison to commercial Targin® tablets.

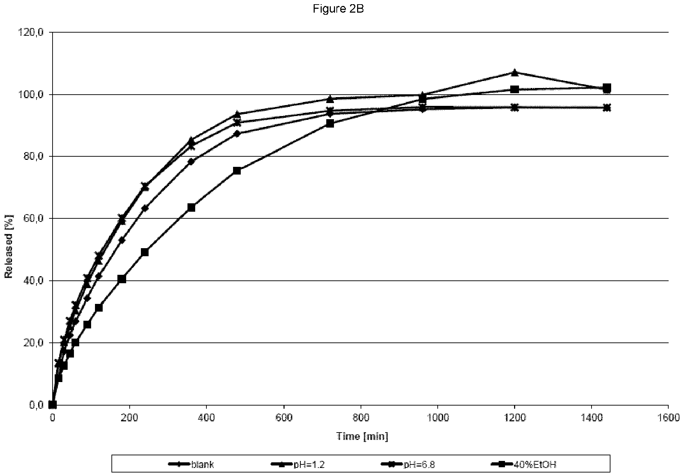

Figure 2B shows the *in vitro* release profile of oxycodone from the tablets as manufactured from composition C-2₅^{oblong} in different release media (blank Fessif, pH 1.2, pH 6.8, and aqueous ethanol 40% (v/v)).

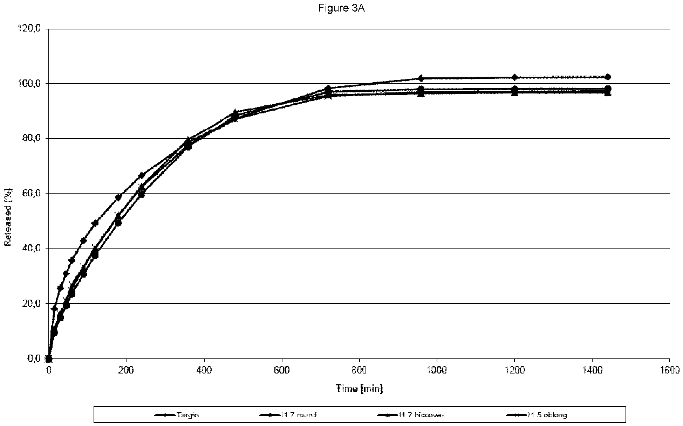

Figure 3A shows the *in vitro* release profile of oxycodone from the tablets I-1₇^{round}, I-1₇^{biconvex}, and I-1₅^{oblong} (different tablet formats) as manufactured from composition I-1 in comparison to commercial Targin® tablets.

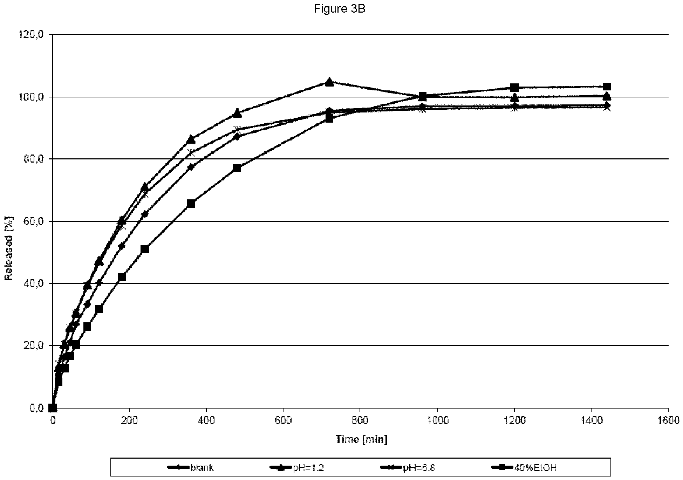

Figure 3B shows the *in vitro* release profile of oxycodone from the tablets as manufactured from composition I-1₅^{oblong} in different release media (blank Fessif, pH 1.2, pH 6.8, and aqueous ethanol 40% (v/v)).

### Analytical tests - content and breaking strength

The content of oxycodone, naloxone and α-tocopherol was analytically quantified in accordance with Ph. Eur. and the breaking strength of the tablets was measured. The results are summarized in the table here below:

| # | Oxycodon | Naloxon | α-Tocopherol | breaking strength |
|---|---|---|---|---|
| C-1₅^{oblong} | 96.5% | 98.25% | 94.50% | > 1000 N |
| C-2₅^{oblong} | 96.2% | 98.28% | 94.75% | > 1000 N |
| I-1₅^{blong} | 95.5% | 97.14% | 94.73% | > 1000 N |

### Analytical tests - attempts of tampering

### a) Tampering in order to render the dosage form administrable by the nasal route

| | |
|---|---|
| Coffee grinder | pulverization |
| Hammer stroke | pulverization, brittle fracture |
| Breaking strength | pulverization |

The tablets were treated by means of o commercially available household coffee mill, type Bosch MKM6000, 180W, Typ KM13. Subsequently, the thus obtained material was analyzed by means of a sieving tower (Haver & Boecker, analysis sieve, diameter 50 mm) equipped with a bottom plate, displacement ring, lid, and 14 sieves the mesh sizes ranging from 0.045 mm to 4.000 mm, namely 0.045 mm; 0.063 mm; 0.090 mm; 0.125 mm; 0.180 mm; 0.250 mm; 0.355 mm; 0.500 mm; 0.710 mm; 1.000 mm; 1.400 mm; 2.000 mm; 2.800 mm; 4.000 mm. The amplitude was set to 1.5 mm.

The hammer stroke test was performed by means of a free falling weight testing device Type 40-550-001, 40-550-011 ff, Coesfeld GmbH & Co. KG, Germany. The following parameters were set:
Falling height: 1000 mm±1%
Falling weight: 500 g±2%
Form of falling weight / impact area: 25 mm x 25 mm
Position of sample: loosely positioned in the center of the sample holder

The measuring result was qualified according to the following scale:
(A) tablet apparently undamaged
(B) tablet has been compressed but is widely undamaged
(C) tablet has been compressed and is lacerated at its edges
(D) tablet has been disrupted into several pieces
(E) tablet has been pulverized

The results are summarized in the table here below:

| Test | Targin^{oblong} | C-1₅^{oblong} | C-2₅^{oblong} | I-1₅^{oblong} |
|---|---|---|---|---|
| Formulation | | | | |
| a) Coffee grinder (2 min); Sieve analysis [µm] (mean 3 x, n=1) | | | | |
| <125 µm: | ∼3% | ∼0% | ∼0% | ∼1% |
| 125-1000 µm: | ∼45% | ∼ 31.5% | ∼ 30% | ∼ 42% |
| >1000 µm: | ∼ 52% | ∼ 68.5% | ∼ 70% | ∼ 57% |
| b) Hammer stroke | (E) | (C) | (C) | (C) |
| c) Breaking strength (n=10) | 87 N (n=10) | > 1000 N (n=9) | > 1000 N (n=7) | > 1000 N (n=8) |
| | Min: 78 N | | | |
| | Max: 100 N | | | |

### b) Tampering in order to render the dosage form administrable by the intravenous route

Boiling for 5 min with 5 mL water (intact tablet, grinded tablet); drawing up with filter and large (G21) needle
The extractable content of pharmacologically active compound was determined by
(i) subjecting a tablet for 5 minutes in 5.0 mL of boiling water,
(ii) supplementing lost water, if any,
(iii) separating the overhead liquid solution from the remainder by means of a syringe, equipped with a cigarette filter and a canula, 0.80 x 40 mm BL/LB; 21 G x 1 1/2", and
(iv) determining the pharmacologically active compound content in the drawn liquid by HPLC analysis.

The following extracted contents were determined:

| [wt.-%] | Targin^{oblong} | C-1₅^{oblong} | C-2₅^{oblong} | I-1₅^{oblong} |
|---|---|---|---|---|
| Oxycodone | 87.7 | 24.1 | 22.6 | 22.5 |
| Naloxone | 77.2 | 21.9 | 21.0 | 21.7 |

An undestroyed tablet was treated for 2 min by a coffee mill. The thus obtained material was boiled in 5 mL water for 5 min and the supernatant liquid was drawn up by means of a syringe, equipped with 21G canula and filter.

The following extracted contents were determined:

| [wt.-%] | Targin^{oblong} | C-1₅^{oblong} | C-2₅^{oblong} | I-1₅^{blong} |
|---|---|---|---|---|
| Oxycodone | 71.4 | 30.1 | 37.6 | 37.8 |
| Naloxone | 52.7 | 31.8 | 36.5 | 36.1 |

Compared to commercial product Targin^{oblong}, substantially less opioid agonist and opioid antagonist could be extracted from the dosage forms C-1₅^{oblong}, C-2₅^{oblong}, and I-1₅^{oblong}. Furthermore, approximately the same amount of opioid agonist and opioid antagonist could be extracted from the dosage forms C-1₅^{oblong}, C-2₅^{oblong}, and I-1₅^{oblong}. From the commercial product Targin^{oblong}, however, more opioid agonist could be extracted than opioid antagonist; this effect is disadvantageous with respect to the avoidance of abuse.

### c) Tampering in order to render the dosage form administrable by the oral route

### Extraction for 30 min with 30 mL of water or 40% Ethanol

Extraction for 30 min with 30 mL of boiling water (tea) [water boils in the beginning and is then allowed to cool down during the 30 min]

| extraction test [wt.-%] | Targin^{oblong} | | C-1₅^{oblong} | | C-2₅^{oblong} | | I-1₅^{blong} | |
|---|---|---|---|---|---|---|---|---|
| | oxy. | nal. | oxy. | nal. | oxy. | nal. | oxy. | nal. |
| intact tablet + 30 ml water (ambient temperature) 30 min | 21.6 | 21.1 | 11.8 | 11.0 | 14.0 | 12.6 | 13.2 | 12.2 |
| intact tablet + 30 ml water/ethanol 40% (v/v) 30 min shaking | 13.9 | 13.8 | 7.9 | 7.2 | 7.6 | 6.7 | 8.4 | 7.5 |
| intact tablet + 30 ml water boiling once 30 min | 93.5 | 88.9 | 42.5 | 40.2 | 42.0 | 39.2 | 40.6 | 39.4 |

| | | | | | | | | |
|---|---|---|---|---|---|---|---|---|
| oxy. = oxycodone nal. = naloxone | | | | | | | | |

### Analytical tests - storage stability

The storage stability of the dosage forms C-1₅^{oblong}, C-2₅^{oblong}, and I-1₅^{oblong} was investigated.

| C-1₅^{oblong} | 0 month | | 3 months 25°C | 3 months 40°C 75%RH |
|---|---|---|---|---|
| oxycodone | 97.1% | | 99.9% | 100.1% |
| Σ oxycodone C+D+N-oxide | 0.06% | | 0.13% | 0.13% |
| naloxone | 98.3% | | 103.3% | 102.2% |
| Σ naloxone impurities | 0.18% | | 0.44% | 0.46% |
| α-tocopherol | 95.5% | | 95.9% | 94.8% |
| breaking strength | >1000 N (n=9) | | >1000 N (n=9) | >1000 N (n=8) |

All assays are normalized with respect to the tablet weight.

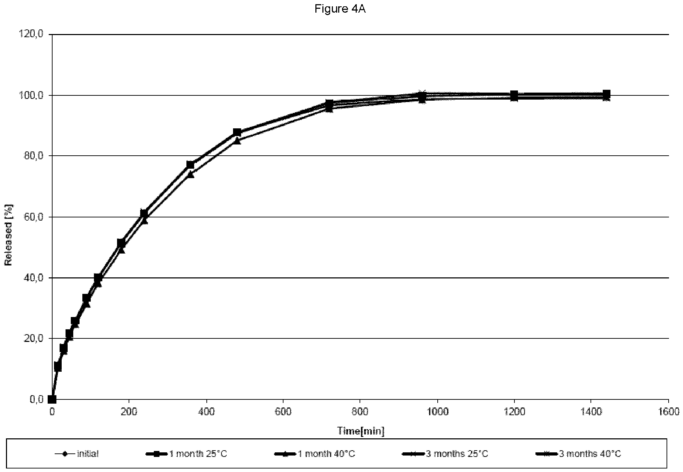

Figure 4A shows the *in vitro* release profile of oxycodone from the tablets C-1₅^{oblong} before and after storage under the various storage conditions.

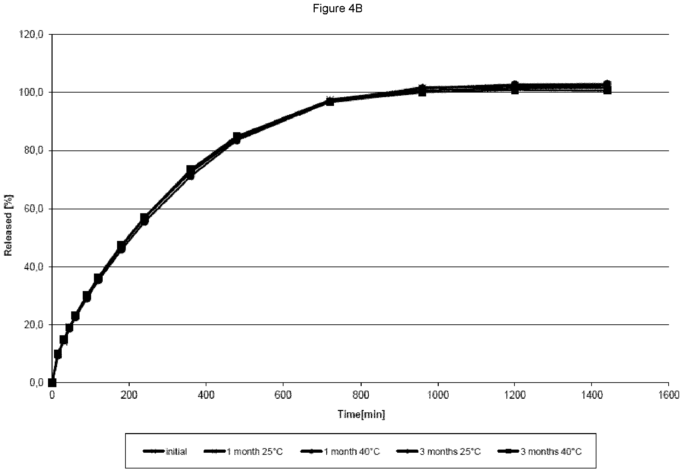

Figure 4B shows the *in vitro* release profile of naloxone from the tablets C-1₅^{oblong} before and after storage under the various storage conditions.

| C-2₅^{oblong} | 0 month | | 3 months 25°C | 3 months 40°C 75%RH |
|---|---|---|---|---|
| oxycodone | 97.3% | | 100.9% | 102.2% |
| Σ oxycodone C+D+N-oxide | 0.06% | | 0.14% | 0.15% |
| naloxone | 98.9% | | 100.7% | 101.2% |
| Σ naloxone impurities | 0.00% | | 0.45% | 0.41% |
| α-tocopherol | 92.9% | | 95.6% | 92.4% |
| breaking strength | >1000 N (n=10) | | >1000 N (n=10) | >1000 N (n=9) |

All assays are normalized with respect to the tablet weight.

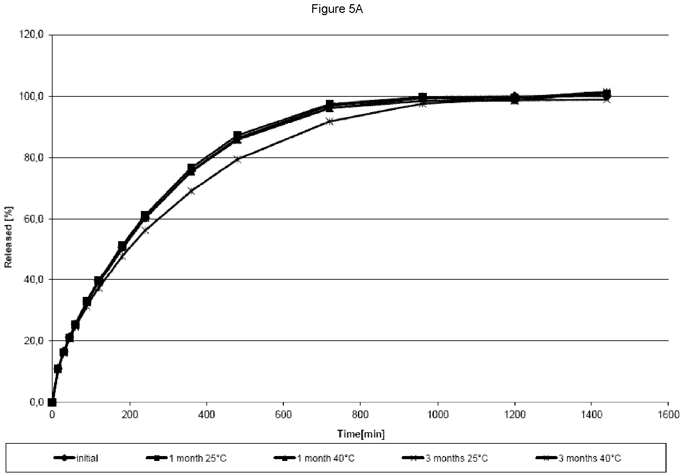

Figure 5A shows the *in vitro* release profile of oxycodone from the tablets C-2₅^{oblong} before and after storage under the various storage conditions.

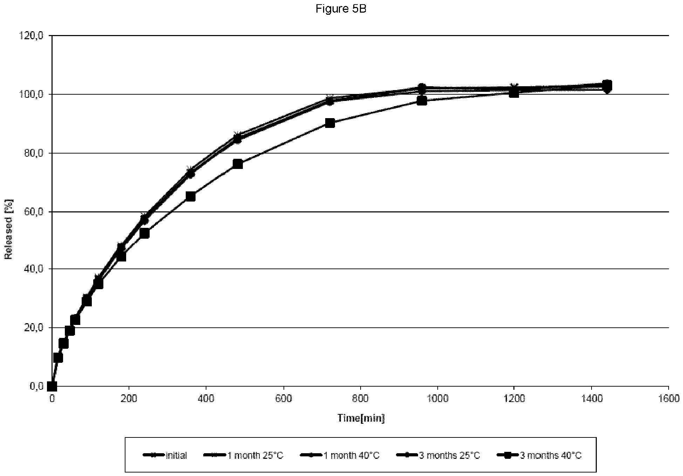

Figure 5B shows the *in vitro* release profile of naloxone from the tablets C-2₅^{oblong} before and after storage under the various storage conditions.

| I-1₅^{blong} | 0 month | | 3 months 25°C | 3 months 40°C 75%RH |
|---|---|---|---|---|
| oxycodone | 96.4% | | 99.1% | 99.0% |
| Σ oxycodone C+D+N-oxide | 0.06% | | 0.15% | 0.14% |
| naloxone | 99.2% | | 100.5% | 101.4% |
| Σ naloxone impurities | 0.00% | | 0.30% | 0.22% |
| α-tocopherol | 93.1% | | 94.4% | 92.8% |
| breaking strength | >1000 N (n=6) | | >1000 N (n=6) | >1000 N (n=4) |

All assays are normalized with respect to the tablet weight.

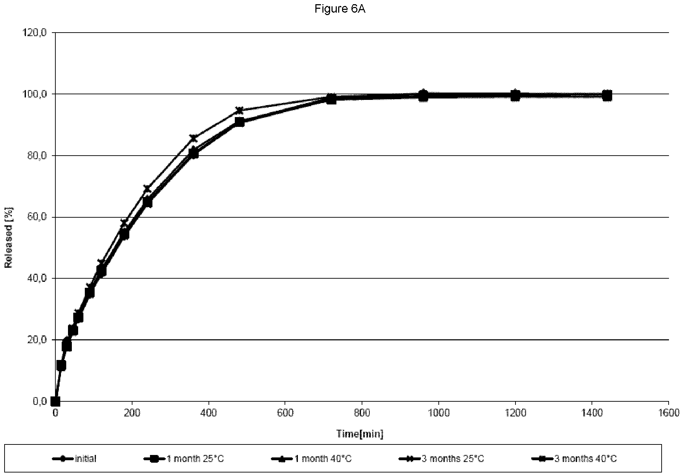

Figure 6A shows the *in vitro* release profile of oxycodone from the tablets I-1₅^{oblong} before and after storage under the various storage conditions.

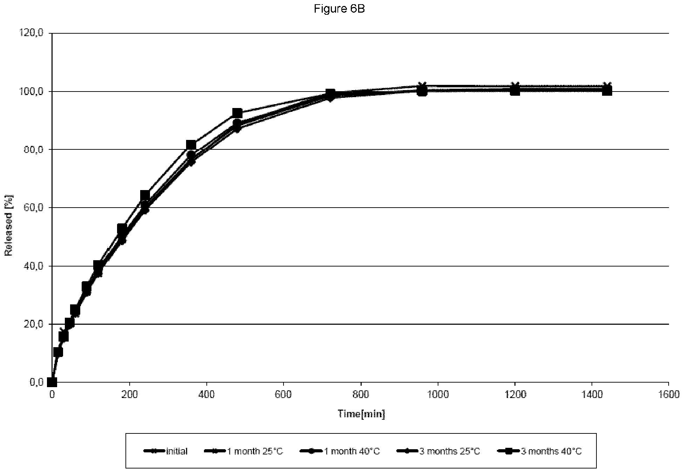

Figure 6B shows the *in vitro* release profile of naloxone from the tablets I-1₅^{oblong} before and after storage under the various storage conditions.

All three tested formulations fulfill the impurity limits given by the API monographs Ph. Eur. (7th edition, 2011) after 3 months of storage at 40°C and 75% RH. However, the dosage form according to the invention I-1₅^{oblong} is clearly the formulation with best stability characteristics within this test period.

### Comparative Example:

### Composition of formulations

Round tablets having a diameter of 10 mm, a radius of curvature of 8 mm and a mass of 333.0 mg were prepared from mixtures of the following components:

| components | # 1 | | #2 | |
|---|---|---|---|---|
| | mg | wt.-% | mg | wt.-% |
| Tramadol HCl | 83.25 | 25.0 | 83.25 | 25.0 |
| Polyethylene oxide Mw 600,000 | 249.75 | 75.0 | - | - |
| Polyethylene oxide Mw 7,000,000 | - | - | 249.75 | 75.0 |

Tramadol HCI and polyethylene oxide were weighed and mixed for 15 minutes in a rolling glass mixer at 14 rpm. Then, the mixtures were compressed at ambient temperature on a Korsch EK0 (punch format 10 mm, radius of curvature 8 mm) at two different pressure forces. The breaking strength was measured by means of a Zwick breaking strength tester (average value over 10 tablets).

The results of the measurements are summarized in the table here below:

| | # 1 | | #2 | |
|---|---|---|---|---|
| | M_{W} 600,000 | | M_{W} 7,000,000 | |
| pressure force [N] | ≈ 7000 | ≈ 40000 | ≈ 5000 | ≈ 38000 |
| breaking strength [N] | **94.2** | **145.1** | **54.0** | **121.1** |

The above experimental data demonstrate that pharmaceutical dosage forms having an increased breaking strength, i.e. a breaking strength of at least 300 N, cannot be obtained by direct compression at room temperature.

However, when performing the same process of preparation at elevated temperature, i.e. when compressing the identical powder mixture at elevated temperature, dosage forms having a breaking strength of at least 300 N can be obtained.

Similar to diamond and graphite, which both consist of pure carbon, the chemical composition does not tell much about the mechanical properties.

### Summary:

The invention relates to a pharmaceutical dosage form for oral administration having a breaking strength of at least 300 N and comprising (i) a pharmacologically active ingredient; (ii) an opioid antagonist and/or an aversive agent; (iii) a polyalkylene oxide having an average molecular weight of at least 200,000 g/mol; and (a) comprising (iv) an anionic polymer; and/or (b) having a storage stability at 40°C of at least 3 months.

## Claims

1. A pharmaceutical dosage form for oral administration having a breaking strength of at least 300 N and comprising
(i) an pharmacologically active ingredient which is an opioid agonist;
(ii) an opioid antagonist which is selected from the group consisting of naltrexone, naloxone, nalmefene, cyclazacine, levallorphan, pharmaceutically acceptable salts thereof and mixtures thereof;
(iii) a polyalkylene oxide having an average molecular weight of at least 200,000 g/mol; and
(iv) an anionic polymer, which is derived from a monomer selected from acrylic acid, alkyl acrylates and alkyl alkacrylates, or a combination thereof;
wherein the pharmacologically active ingredient and the opioid antagonist are embedded in a prolonged release matrix comprising the polyalkylene oxide and the anionic polymer.

2. The pharmaceutical dosage form according to claim 1, wherein the anionic polymer is an optionally cross-linked homopolymer of acrylic acid.

3. The pharmaceutical dosage form according to claim 1, wherein the anionic polymer is an optionally cross-linked copolymer of acrylic acid and C₁₀-C₃₀-alkyl acrylate.

4. The pharmaceutical dosage form according to claim 1, wherein the anionic polymer is an interpolymer, namely an optionally cross-linked homopolymer of acrylic acid; or an optionally cross-linked copolymer of acrylic acid and C₁₀-C₃₀-alkyl acrylate; which contain a block copolymer of polyethylene glycol and a C₈-C₃₀-alkyl acid.

5. The pharmaceutical dosage form according to any of the preceding claims, wherein in accordance with Ph. Eur. the *in vitro* release profile of the pharmacologically active ingredient essentially corresponds to the *in vitro* release profile of the opioid antagonist.

6. The pharmaceutical dosage form according to any of the preceding claims, wherein the pharmacologically active ingredient and the opioid antagonist are homogeneously distributed over the pharmaceutical dosage form or, when the pharmaceutical dosage form comprises a film coating, over the coated core of the pharmaceutical dosage form.

7. The pharmaceutical dosage form according to any of the preceding claims, which is configured for administration once daily or twice daily.

8. The pharmaceutical dosage form according to any of the preceding claims, which is monolithic.

9. The pharmaceutical dosage form according to any of the preceding claims, wherein the content of the polyalkylene oxide is at least 30 wt.-%, based on the total weight of the pharmaceutical dosage form.

10. The pharmaceutical dosage form according to any of the preceding claims, wherein the content of anionic polymer is within the range of 5.0±4.5 wt.-%, based on the total weight of the pharmaceutical dosage form.

11. The pharmaceutical dosage form according to any of the preceding claims, which is thermoformed.

12. The pharmaceutical dosage form according to claim 11, which is hot-melt extruded.

13. The pharmaceutical dosage form according to any of the preceding claims, which is tamper-resistant.

14. The pharmaceutical dosage form according to any of the preceding claims, wherein the pharmacologically active ingredient is oxycodone or a physiologically acceptable salt thereof.

15. The pharmaceutical dosage form according to any of the preceding claims, which contains
- a plasticizer selected from the group consisting of polyalkylene glycol, triacetin, fatty acids, fatty acid esters, waxes and/or microcrystalline waxes; and/or
- an antioxidant selected from the group consisting of ascorbic acid, α-tocopherol, butylhydroxyanisol, butylhydroxytoluene, salts of ascorbic acid, ascorbylic palmitate, monothioglycerine, coniferyl benzoate, nordihydroguajaretic acid, gallus acid esters, phosphoric acid, vitamin E-succinate, vitamin E-palmitate and/or sodium bisulphite.

## Patentansprüche

1. Pharmazeutische Dosisform zur oralen Verabreichung mit einer Bruchfestigkeit von mindestens 300 N, Folgendes umfassend:
(i) einen pharmakologisch aktiven Wirkstoff, welcher ein Opioid-Agonist ist;
(ii) einen Opioid-Antagonisten, der aus der Gruppe ausgewählt ist, die aus Naltrexon, Naloxon, Nalmefen, Cyclazocin, Levallorphan, pharmakologisch annehmbaren Salzen davon und Mischungen daraus besteht;
(iii) ein Polyalkylenoxid mit einem mittleren Molekulargewicht von mindestens 200.000 g/mol; und
(iv) ein anionisches Polymer, das von einem Monomer gewonnen wird, das aus Acrylsäure, Alkylacrylaten und Alkylalkacrylaten oder Kombinationen davon ausgewählt ist;
wobei der pharmakologisch aktive Wirkstoff und der Opioid-Antagonist in einer Matrix mit verlängerter Freisetzung eigebettet sind, die das Polyalkylenoxid und das anionische Polymer umfasst.

2. Pharmazeutische Dosisform nach Anspruch 1, wobei das anionische Polymer ein gegebenenfalls vernetztes Homopolymer von Acrylsäure ist.

3. Pharmazeutische Dosisform nach Anspruch 1, wobei das anionische Polymer ein gegebenenfalls vernetztes Copolymer von Acrylsäure und C₁₀-C₃₀-Alkylacrylat ist.

4. Pharmazeutische Dosisform nach Anspruch 1, wobei das anionische Polymer ein Interpolymer ist, nämlich ein gegebenenfalls vernetztes Homopolymer von Acrylsäure; oder ein gegebenenfalls vernetztes Copolymer von Acrylsäure und C₁₀-C₃₀-Alkylacrylat, welches eine Blockcopolymer von Polyethylenglykol und einer C₈-C₃₀-Alkylsäure beinhaltet.

5. Pharmazeutische Dosisform nach einem der vorstehenden Ansprüche, wobei gemäß Ph. Eur. das in-vitro Freisetzungsprofil des pharmakologisch aktiven Wirkstoffs im Wesentlichen dem in-vitro Freisetzungsprofil des Opioid-Antagonisten entspricht.

6. Pharmazeutische Dosisform nach einem der vorstehenden Ansprüche, wobei der pharmakologisch aktive Wirkstoff und der Opioid-Antagonist homogen über die pharmazeutische Dosisform oder, wenn die pharmakologische Dosisform eine Filmbeschichtung umfasst, über dem beschichteten Kern der pharmakologischen Dosisform verteilt sind.

7. Pharmazeutische Dosisform nach einem der vorstehenden Ansprüche, welche für eine Verabreichung einmal täglich oder zweimal täglich konfiguriert ist.

8. Pharmazeutische Dosisform nach einem der vorstehenden Ansprüche, welche monolithisch ist.

9. Pharmazeutische Dosisform nach einem der vorstehenden Ansprüche, wobei der Gehalt von Polyalkylenoxid mindestens 30 Gew.-%, basierend auf dem Gesamtgewicht der pharmazeutischen Dosisform, beträgt.

10. Pharmazeutische Dosisform nach einem der vorstehenden Ansprüche, wobei der Gehalt von anionischem Polymer in dem Bereich von 5,0 ± 4,5 Gew.-%, basierend auf dem Gesamtgewicht der pharmazeutischen Dosisform, liegt.

11. Pharmazeutische Dosisform nach einem der vorstehenden Ansprüche, welche thermogeformt ist.

12. Pharmazeutische Dosisform nach Anspruch 11, welche heißschmelzend extrudiert ist.

13. Pharmazeutische Dosisform nach einem der vorstehenden Ansprüche, welche gegen Missbrauch gesichert ist.

14. Pharmazeutische Dosisform nach einem der vorstehenden Ansprüche, wobei der pharmakologisch aktive Wirkstoff Oxycodon oder ein physiologisch annehmbares Salz davon ist.

15. Pharmazeutische Dosisform nach einem der vorstehenden Ansprüche, welche Folgendes enthält:
- einen Weichmacher, der aus der Gruppe ausgewählt ist, die aus Polyalkylenglycol, Triacetin, Fettsäuren, Fettsäureestern, Wachsen und/oder mikrokristallinen Wachsen besteht; und/oder
- ein Antioxidationsmittel, das aus der Gruppe ausgewählt ist, die aus Ascorbinsäure, α-Tocopherol, Butylydroxyanisol, Butylhydroxytoluol, Salze von Ascorbinsäure, Ascorbylpalmitat, Monothioglycerin, Coniferylbenzonat, nordihydroguaiaretische Säure, Gallussäureestern, Phosphorsäure, Vitamin E-Succinat, Vitamin E-Palminat und oder Natriumbisulfit besteht.

## Revendications

1. Forme pharmaceutique pour administration orale présentant une résistance à la rupture d'au moins 300 N et comprenant
(i) un ingrédient pharmacologiquement actif qui est un agoniste opioïde ;
(ii) un antagoniste opioïde qui est sélectionné dans le groupe constitué de la naltrexone, de la naloxone, du nalméfène, de la cyclazacine, du lévallorphane, des sels pharmaceutiquement acceptables de ceux-ci et des mélanges de ceux-ci ;
(iii) un polyoxyde d'alkylène présentant un poids moléculaire moyen d'au moins 200 000 g/mol ; et
(iv) un polymère anionique, qui est dérivé d'un monomère sélectionné parmi l'acide acrylique, les acrylates d'alkyle et les alkacrylates d'alkyle, ou une combinaison de ceux-ci ;
dans laquelle l'ingrédient pharmacologiquement actif et l'antagoniste opioïde sont enrobés dans une matrice à libération prolongée comprenant le polyoxyde d'alkylène et le polymère anionique.

2. Forme pharmaceutique selon la revendication 1, dans laquelle le polymère anionique est un homopolymère d'acide acrylique facultativement réticulé.

3. Forme pharmaceutique selon la revendication 1, dans laquelle le polymère anionique est un copolymère d'acide acrylique et d'une acrylate d'alkyle en C₁₀ à C₃₀ facultativement réticulé.

4. Forme pharmaceutique selon la revendication 1, dans laquelle le polymère anionique est un interpolymère, c'est-à-dire un homopolymère d'acide acrylique facultativement réticulé ; ou un copolymère d'acide acrylique et d'une acrylate d'alkyle en C₁₀ à C₃₀ facultativement réticulé ; qui contient un copolymère séquencé de polyéthylène glycol et d'un (alkyle en C₈ à C₃₀)-acide.

5. Forme pharmaceutique selon l'une quelconque des revendications précédentes, dans laquelle conformément à la Ph. Eur. le profil de libération *in vitro* de l'ingrédient pharmacologiquement actif correspond essentiellement au profil de libération *in vitro* de l'antagoniste opioïde.

6. Forme pharmaceutique selon l'une quelconque des revendications précédentes, dans laquelle l'ingrédient pharmacologiquement actif et l'antagoniste opioïde sont répartis de manière homogène sur la forme pharmaceutique ou, quand la forme pharmaceutique comprend un pelliculage, sur le noyau pelliculé de la forme pharmaceutique.

7. Forme pharmaceutique selon l'une quelconque des revendications précédentes, qui est configurée pour une administration une fois par jour ou deux fois par jour.

8. Forme pharmaceutique selon l'une quelconque des revendications précédentes, qui est monolithique.

9. Forme pharmaceutique selon l'une quelconque des revendications précédentes, dans laquelle la teneur en polyoxyde d'alkylène est au moins de 30 % en poids sur la base du poids total de la forme pharmaceutique.

10. Forme pharmaceutique selon l'une quelconque des revendications précédentes, dans laquelle la teneur en polymère anionique est située dans la plage de 5,0 ± 4,5 % en poids, sur la base du poids total de la forme pharmaceutique.

11. Forme pharmaceutique selon l'une quelconque des revendications précédentes, qui est thermoformée.

12. Forme pharmaceutique selon la revendication 11, qui est extrudée à l'état fondu.

13. Forme pharmaceutique selon l'une quelconque des revendications précédentes, qui est inviolable.

14. Forme pharmaceutique selon l'une quelconque des revendications précédentes, dans laquelle l'ingrédient pharmacologiquement actif est l'oxycodone ou un sel physiologiquement acceptable de celle-ci.

15. Forme pharmaceutique selon l'une quelconque des revendications précédentes, qui contient
- un plastifiant sélectionné dans le groupe constitué d'un polyalkylène glycol, de la triacétine, des acides gras, des esters d'acide gras, des cires et/ou des cires microcristallines ; et/ou
- un antioxydant sélectionné dans le groupe constitué de l'acide ascorbique, de l'α-tocophérol, du butylhydroxy-anisol, du butylhydroxytoluène, des sels d'acide ascorbique, du palmitate ascorbylique, de la monothio-glycérine, du benzoate de coniféryle, de l'acide nordihydroguajarétique, des esters d'acide gallus, de l'acide phosphorique, du succinate de vitamine E, du palmitate de vitamine E et/ou du bisulfite de sodium.
